(19) Europäisches Patentamt / European Patent Office / Office européen des brevets

(11) **EP 3 494 204 B1**

(12) **EUROPÄISCHE PATENTSCHRIFT**

(45) Veröffentlichungstag und Bekanntmachung des Hinweises auf die Patenterteilung:
**29.04.2020  Patentblatt 2020/18**

(21) Anmeldenummer: **18709998.1**

(22) Anmeldetag: **05.03.2018**

(51) Int Cl.:
**C12M 1/107** (2006.01)          **C12M 1/34** (2006.01)
**C12M 1/36** (2006.01)

(86) Internationale Anmeldenummer:
**PCT/EP2018/055378**

(87) Internationale Veröffentlichungsnummer:
**WO 2018/162429 (13.09.2018 Gazette 2018/37)**

(54) **VERFAHREN ZUM BETREIBEN EINER BIOGASANLAGE**

METHOD FOR OPERATING A BIOGAS PLANT

PROCÉDÉ POUR FAIRE FONCTIONNER UNE INSTALLATION DE BIOGAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.03.2017  DE 102017104642**

(43) Veröffentlichungstag der Anmeldung:
**12.06.2019  Patentblatt 2019/24**

(73) Patentinhaber: **Agraferm GmbH**
**85276 Pfaffenhofen (DE)**

(72) Erfinder: **KUBE, Jürgen**
**Guildford GU2 9LF (GB)**

(74) Vertreter: **HGF Europe LLP**
**Neumarkter Str. 18**
**81673 München (DE)**

(56) Entgegenhaltungen:
WO-A1-2012/175701          WO-A1-2014/075850
WO-A2-2009/076947          DE-A1- 4 428 818
DE-A1-102006 053 863          DE-A1-102012 009 640
DE-A1-102013 004 079          DE-A1-102015 107 224
DE-B3-102007 012 861

EP 3 494 204 B1

# EP 3 494 204 B1

## Beschreibung

Die vorliegende Erfindung betrifft ein Verfahren zum Betreiben einer Biogasanlage und insbesondere einer Biogasaufbereitungsanlage nach dem Oberbegriff des Anspruchs 1.

Biogasanlagen sind seit langem bekannt und erlangen in Deutschland im Rahmen der Energiewende eine erhebliche Bedeutung.

Bei der Herstellung von Biogas werden organische Substrate unter Abwesenheit von Sauerstoff vergoren. Bei der Vergärung entsteht Biogas. Biogas ist eine Mischung aus überwiegend Methan und $CO_2$ aus der Vergärung. Dieses Biogas kann vor Ort in Blockheizkraftwerken oder Brennern energetisch verwertet werden. Soll die Nutzung jedoch abseits der Erzeugung erfolgen, ist eine Aufreinigung des Biogases zu Biomethan und Einspeisen des Biomethans in ein Gasnetz oder einen Speicher notwendig. Die Anlagen, in denen das $CO_2$ und teilweise andere Begleitgase abgetrennt werden heißen Biogasaufbereitungsanlagen.

Für die Aufreinigung von Biogas zu Biomethan sind verschiedene Verfahren bekannt und werden in der Praxis erfolgreich umgesetzt. Bekannt sind Wasserwäschen, organische Wäschen (z.B. mit Genosorb oder Amin-basierten Lösemitteln), Druckwechseladsorptionen und Membranbasierte Gaspermeationsverfahren. In Deutschland hat sich speziell das Fraunhofer Umsicht-Institut mit der Biogaseinspeisung beschäftigt und vergleichende Studien veröffentlicht.

Die wichtigen Kennzahlen der Aufbereitungstechnik sind neben der Verfügbarkeit der Energiebedarf der Anlage und der Methanschlupf. Der Energiebedarf der Anlage wird in kWh/Nm³ Rohbiogas angegeben und beträgt bei Biogas mit 50% Methan ca. 0,20 bis 0,30 kWh/Nm³. Wenn das Methan nicht vollständig aus dem Biogas abgetrennt werden kann, ist es manchmal notwendig, eine Mindestkonzentration Methan im Schwachgas einzuhalten, um die restlichen Methanmengen thermisch zu oxidieren. Energiebedarf und Methanschlupf hängen von der Art des Verfahrens aber auch von Genehmigungsauflagen und der Betriebsweise ab. Der Methanschlupf der Anlagen beträgt null bei organischen Wäschen und kryogenen Membranverfahren und 0,5 bis 1% bei allen anderen modernen Verfahren.

Besonders in den letzten Jahren hat sich das Gaspermeationsverfahren immer mehr durchgesetzt. Es ist einfacher zu beherrschen als die meisten Wäschen und kann daher von einer Vielzahl an Marktteilnehmern vertrieben werden. Es ist zudem nach unten problemlos skalierbar, sehr kompakt und verlangt keine hohen Kolonnen. Auch die Wasserwäschen konnten ihren Marktanteil ausbauen, speziell bei Biogasanlagen, die Abfälle vergären sind Wasserwäschen sehr robust gegenüber Begleitgasen aus dem Abfall-Biogas.

Bereits 1990 wurde in der Dissertation "Untersuchungen zur Methananreicherung von Biogas durch Gaspermeation" von Heinz Erhard Ehresmann an der RWTH Aachen die Möglichkeit zur Gewinnung einer brennwertreichen Gasfraktion aus Biogas untersucht.

Viele Entwicklungen der letzten Jahre auf dem Gebiet der Gaspermeationen wurden von Firmen und Forschern im Raum Wien/Österreich entwickelt. Zwei der ersten kommerziellen Gaspermeationsanlagen wurden in Bruck an der Leitha 2006 und in Magarethen am Moos bei Wien 2007 in Betrieb genommen.

Ende Februar 2012 wurde die erste Biogasaufbereitungsanlage in Großbritannien mit einer dreistufigen Gaspermeationsanlage in Betrieb genommen. Die Anlage produziert 400 Nm³/h Biomethan.

Die WO 2012/000727 A1 beschreibt ein dreistufiges Gaspermeationsverfahren, bei dem das Rohbiogas in einer ersten Stufe in ein Retentat und Permentat getrennt wird, die beiden Ströme jeweils einer weiteren Stufe zugeführt werden, das Permeat der Retentatstufe und das Retentat der Permeatstufe wieder mit dem Rohbiogas vermischt werden und eine Vakuumpumpe auf der Permeatseite der Retentatstufe installiert ist. Dieses Verfahren wird in der Praxis häufig verwendet und wurde in der Vergangenheit an viele Hersteller lizensiert.

Die DE 10 2013 004 079 A1 beschreibt ein dreistufiges Gaspermeationsverfahren nach der WO 2012/000727 A1 bei der die Vakuumpumpe nach dem Methangehalt im Produktgas geregelt wird. In einer besonderen Ausführungsform wird zusätzlich der Retentatdruck der Permeatstufe nach dem Methangehalt des Schwachgases geregelt. Die Regelung der Vakuumpumpe nach dem Methangehalt im Produktgas hat zwei wesentliche Nachteile. Erstens liegt der Methangehalt im Produktgas zwischen 95% und 99% und kann wesentlich ungenauer gemessen werden als der $CO_2$-Anteil im Produktgas, der zwischen 0,5 und 3,5% liegt. Zweitens berücksichtigt die Reglung nicht die Anwesenheit der inerten Begleitgase Stickstoff und Sauerstoff, die mit der Vakuumpumpe nur unwesentlich abgeschieden werden können. Eine 98%ige Methankonzentration im Produktgas ist sehr gut, wenn 1,5% $O_2$ und $N_2$ vorliegen, aber noch verbesserungswürdig, wenn nur 0,5% $N_2$ und $O_2$ vorliegen. Wie später gezeigt werden wird, sind auch andere Lösungen möglich, indem z.B. die Vakuumpumpe auf einen festen, möglichst geringen Druck geregelt wird und stattdessen der Kompressordruck (Retentatdruck der Stufe 2) als Stellgröße für die Einhaltung der Methan- oder besser $CO_2$-Konzentration im Produktgas verwendet wird.

Die WO 2014/075850 A1 beschreibt ein dreistufiges Gaspermeationsverfahren nach der WO 2012/000727 A1, bei dem das Rohbiogas in einer ersten Stufe in ein Retentat und Permentat getrennt wird, die beiden Ströme jeweils einer weiteren Stufe zugeführt werden, der Druck des Permeats der Retentatstufe und des Retentats der Permeatstufe kontrolliert wird und die beiden Ströme wieder mit dem Rohbiogas vermischt werden. Es wird weiterhin eine Regeleinrichtung beschrieben, bei der die Konzentration einer Gaskomponente im Produkt-oder Schwachgas die Regelgröße

2

ist und der Druck im System die Stellgröße ist, um die Produkt- und Schwachgasqualitäten auf einen Sollwert zu regeln. Die Stellglieder sind zum Beispiel die Vakuumpumpe auf der Permeatseite der zweiten Stufe und/oder das Drosselventil auf der Retentatseite der dritten Stufe. In einer bevorzugten Ausführungsform sind nicht die Konzentrationen die Regelgröße sondern die einfacher zu messenden Flüsse. Das System wird einmal kalibriert und die Stellgrößen anhand der Flüsse berechnet. Die aufwändige und meist diskontinuierliche Qualitätsmessung im Gas entfällt. Würden die Membranen altern, müsste man die Anlage neu kalibrieren. Die vorgeschlagene Kalibrierung betrachtet nicht die Temperaturen im Prozess.

In der WO 2012 2012/175701 A1 ist ein Verfahren zur Biogaserzeugung offenbart, bei dem in einem Simulationsmodell der biologische Prozess in einer Biogasanlage simuliert wird. Eingangsgrößen werden in dem Simulationsmodell vorgegeben. In dem Simulationsmodell werden daraufhin die Ausgangsgrößen berechnet. Durch Verändern einer Eingangsgröße können verschiedene Ausgangsgrößen berechnet werden. Dadurch kann ein lokales Optimum berechnet werden. Wird ein solches Optimum gefunden werden die entsprechenden Parameter in der Biogasanlage eingestellt. Durch die Methode können Ausgangsgrößen abgeschätzt werden, bevor sie eingestellt werden. Nachteilig dieser Methode ist Komplexität des Modells. Ein Simulationsmodell kann nur in einem engen Parameterbereich zuverlässig die Ausgangsgrößen simulieren.

Aus der DE 10 2006 053 863 A1 geht ein Verfahren zum Betreiben einer Biogasanlage hervor, in der mit einem Simplex-Verfahren die Betriebsgrößen in der Biogasanlage optimiert werden. Dazu werden ein oder mehrere Betriebsgrößen initial eingestellt. Anschließend werden ein oder mehrere Parameter bestimmt und die Betriebsgrößen anhand eines gewichteten Gütemaßes durch das Simplex-Verfahren optimiert. Die so optimierten Betriebsgrößen werden nun eingestellt und das Verfahren wiederholt. Die Differenz der Betriebsgrößen zwischen zwei Abläufen kann sehr groß sein, wodurch die Optimierung nur sehr langsam konvergiert.

Nachfolgend werden die Begriffe noch einmal definiert.

Biogas ist eine Mischung aus überwiegend Methan und $CO_2$, die in Abwesenheit von Sauerstoff durch Vergärung organischer Substrate gebildet wird. Eine Biogasanlage ist eine Anlage zur Erzeugung von Biogas aus organischen Substraten. Eine Biogasaufbereitungsanlage ist eine Anlage zur Trennung von Biogas in seine Komponenten, insbesondere zur Gewinnung eines fast reinen Methanstroms mit dem Zweck der Einspeisung in ein Gasnetz, zur Nutzung im Mobilitätsmarkt oder zur stofflichen Nutzung des Methans. Der außerdem anfallende $CO_2$-Strom stellt keinen großen Wert dar und wird meist in die Umgebung entlassen. Auch das Gas, welches aus Deponien oder Faultürmen auf Kläranlagen freigesetzt wird, kann als Biogas bezeichnet werden und kann mit dem erfindungsgemäßen Verfahren behandelt werden. In der Literatur wird als Biogas manchmal auch das gasförmige Reaktionsprodukt der thermischen Umsetzung von holzartigen Substraten bezeichnet. Dieses Biogas besteht aus einer Vielzahl an unterschiedlichen Gasen die in eine brennwertreiche und eine brennwertarme Fraktion getrennt werden können. Obwohl das erfindungsgemäße Verfahren auch auf solche Prozesse angepasst werden kann, sollen sie nicht im Vordergrund stehen.

Unabhängige Variable sind Variablen, die verändert werden können, ohne dass sich eine Änderung einer anderen unabhängigen Variable ergibt. Eine abhängige Variable kann nicht frei verändert werden. Unabhängige Variablen werden auch als Freiheitsgrade bezeichnet. Eine unabhängige Variable kann zu einer abhängigen Variablen werden, wenn sie zur Stellgröße eines Reglers wird, der eine Nebenbedingung einhält. Die Unterscheidung in abhängige und unabhängige Variable ist nicht immer trivial. Wird zum Beispiel bei einer Biogasaufbereitungsanlage der Produktgasstrom und die Produkt- und Schwachgasqualität vorgegeben, ist der Inputgasstrom keine unabhängige Variable mehr, da er sich aus der Stoffbilanz um die Anlage ergibt.

Steuerparameter sind Parameter, die ggf. mit einem Sensor gemessen und mit einem Stellglied unmittelbar verändert werden können. Beispiele für einen Steuerparameter sind Temperatur eines Stoffstromes hinter einem Wärmetauscher und Druck eines Stoffstromes hinter einem Kompressor oder Drosselventil. Steuerparameter können abhängige oder unabhängige Variablen sein. Unabhängige Steuerparameter werden durch das erfindungsgemäße Verfahren beeinflusst. Abhängige Steuerparameter werden durch lokale Regler kontrolliert.

Der Zielparameter ist die zu optimierende Größe. Der Zielparameter kann gemessen werden und hängt von den Steuerparametern ab. Der funktionale Zusammenhang zwischen den Steuerparametern und dem Zielparameter ist meist sehr komplex und nicht bekannt. Beispiele für Zielparameter sind Leistungsaufnahme oder Durchsatz der Biogasaufbereitungsanlage sowie Betriebskosten.

Qualitätsparameter sind Größen, die bei der erfindungsgemäßen Optimierung eingehalten werden müssen. Qualitätsparameter können gemessen werden und hängen von den Steuerparametern ab, der funktionale Zusammenhang ist jedoch meist nicht bekannt. Beispiele für Qualitätsparameter sind Gasqualitäten, Leistungsaufnahmen von limitierenden Aggregaten oder Input- oder Output-Ströme.

Ein Flash-Kessel ist ein verfahrenstechnischer Apparat, in dem ein Eingangsstrom sich unter isobaren und isothermen Bedingungen in eine Gas und Flüssigphase teilt, die im thermodynamischen Gleichgewicht zueinander stehen.

Als Purge-Strom wird in der Verfahrenstechnik ein Strom bezeichnet, der aus einem Kreislauf ausgeschleust wird, um ein Akkumulieren von Inertstoffen in diesem Kreislauf zu verhindern, bzw. zu reduzieren.

Eine Auslenkung eines Parameters ist grundsätzlich lokal beschränkt, d.h., dass der Parameter nur innerhalb seiner Umgebung (im Sinne der Topologie) ausgelenkt wird. Dies bedeutet in der Praxis, dass ein Parameter nicht mehr als um einen vorbestimmten Schwellenwert ausgelenkt werden kann. Dieser Schwellenwert beträgt vorzugsweise 10% des jeweiligen Parameterwertes, insbesondere 7,5% bzw. 5% des jeweiligen Parameterwertes.

Gegenüber dem bekannten Verfahren hat die Erfindung die Aufgabe eine Biogasanlage zur Verfügung zu stellen, welche eine Aufbereitungsanlage umfasst, bei der Biogas zu Biomethan und Kohlendioxid aufbereitet wird, wobei die Anlage möglichst energieeffizient betrieben wird und gleichzeitig die Qualitätsparameter erreicht bzw. eingehalten werden.

Die Aufgabe wird mit einem Verfahren mit den Merkmalen des Anspruchs 1 gelöst.

Vorteilhafte Weiterbildungen sind in Unteransprüchen gekennzeichnet. Beim erfindungsgemäßen Verfahren zum Betreiben einer Biogasanlage wird diese schrittweise bezüglich eines Zielparameters optimiert. In einem Optimierungsschritt nach einem Gradienten-basierten Verfahren wird zumindest ein Steuerparameter ausgelenkt und die durch die Auslenkung verursachte Änderung des Zielparameters gemessen. In Abhängigkeit der gemessenen Änderung des Zielparameters wird ein Optimierungsschritt berechnet, bei dem Werte für einstellbare Steuerparameter derart bestimmt werden, dass durch Einstellen der berechneten Steuerparameter an auf diese Werte der Zielparameter der Biogasanlage in Richtung eines vorgegebenen Zieles optimiert wird. Die berechneten Werte der Steuerparameter werden an der Biogasanlage eingestellt. Dieser Optimierungsschritt wird mehrfach wiederholt.

Eine Biogasanlage ist eine komplexe Anlage, bei welcher einer Vielzahl von Steuerparameter einzustellen sind, die sich gegenseitig beeinflussen. Es ist daher schwierig, eine solche Biogasanlage manuell optimal einzustellen. Das erfindungsgemäße Verfahren erlaubt grundsätzlich eine gesamte Biogasanlage oder Teile einer Biogasanlage automatisch optimal einzustellen.

Das Verfahren wird in der Regel für einen oder mehrere Teile einer Biogasanlage, wie z. B. einer Biogasaufbereitungsanlage, einer Biogasaufreinigungsanlage, eine Power-to-Gas-Anlage, eine Druckwasserwäsche, eine organische Wäsche oder eine Druckwechseladsorption verwendet. Diese Teile der Biogasanlage sind mittels eines oder mehrerer Steuerparameter einstellbar, die eine schnelle Reaktion bewirken. Schnell bedeutet, innerhalb einiger Minuten bis maximal 1 Stunde. Vorzugsweise dauert die Reaktion nicht länger als eine halbe Stunde bzw. nicht länger als 15 Minuten. In Biogasanlagen gibt es auch Steuerparameter, wie z. B. die Drehzahl eines Rührwerkes, welche bei einer Veränderung eine Reaktion ausführen, die sich erst nach einigen Tagen oder Wochen auf einen Endwert einstellt. Theoretisch könnten auch derartige langsam reagierende Steuerparameter für das erfindungsgemäße Verfahren verwendet werden. Jedoch würde dann das Verfahren einige Wochen oder Monate dauern, was in der Praxis nicht zielführend ist. Deshalb ist das erfindungsgemäße Verfahren vor allem zum Auslenken von Steuerparametern geeignet, welche eine schnelle Reaktion bewirken.

Da die Steuerparameter ausgelenkt werden, werden sie nur geringfügig verändert, so dass sich der Zielparameter hierauf schnell einstellen kann. Dies erlaubt eine schnelle Messung des veränderten Zielparameters und damit eine schnelle Berechnung des Optimierungschrittes. Die einzelnen Optimierungsschritte können so schnell ausgeführt werden.

Die Steuerparameter sind vorzugsweise unabhängige Variablen. Unabhängige Variablen sind Variablen, die verändert werden können, ohne dass sich eine Änderung einer anderen unabhängigen Variable ergibt. Im Gegensatz hierzu kann eine abhängige Variable nicht frei verändert werden, ohne dass dies unmittelbar Einfluss auf eine andere unabhängige Variable hat. Ein Steuerparameter wird abhängig, wenn er zur Einhaltung eines Qualitätsparameters mittels eines lokalen Reglers eingesetzt wird.

Die Zielfunktion kann beispielsweise die Optimierung des Durchsatzes der Anlage oder die Optimierung der Leistungsaufnahme sein. Es können jedoch auch andere sinnvolle Zielfunktionen gewählt werden, wie z. B. der Erlös der Anlage.

Bei jedem Optimierungsschritt können auch mehrere Steuerparameter ausgelenkt werden.

Das Optimierungsverfahren wird vorzugsweise vollautomatisch ausgeführt, d. h., dass die Steuerparameter von einer Steuereinrichtung gesteuert selbsttätig mittels entsprechender Stellelement ausgelenkt, von der Steuereinrichtung berechnet und an den entsprechenden Stellelementen eingestellt werden.

Der Optimierungsschritt wird vorzugsweise so oft wiederholt, bis sich keine wesentliche Änderung bei den Zielparametern ergibt, womit man ein lokales Optimum gefunden hat.

Bei jedem Optimierungsschritt können die partiellen Ableitungen des Zielparameters nach den Steuerparametern bestimmt werden. Diese partiellen Ableitungen werden dann zur Berechnung von neuen Steuerparametern verwendet.

Um die Suche nach dem lokalen Optimum abzukürzen, kann die ungefähre Lage eines Optimums, bevorzugt des globalen Optimums bekannt sein. Das globale Optimum kann beispielsweise durch eine mechanistische Modellierung des zu optimierenden Prozesses mit anschließender Optimierung gefunden werden. Da ein Modell die Realität nie genau vorhersagt, ist eine anschließende Optimierung in der laufenden Anlage stets vorteilhaft. Ist die ungefähre Lage des globalen Optimums bekannt, muss die anschließende Optimierungsroutine nur noch das lokale Optimum finden.

Die reale Anlage kann außerdem altern, verschleißen oder durch Nachbesserungen modifiziert werden. Diese Änderungen werden in der Regel mit dem Modell nicht erfasst.

Die Steuerparameter können mit entsprechenden Sensoren gemessen werden und die Stellelemente können nach Maßgabe der entsprechenden Messwerte betätigt werden, um die bei den Optimierungsschritten berechneten Werte für die Steuerparameter einzustellen.

Als Optimierungsalgorithmus wird ein Gradienten-basiertes Verfahren verwendet. Zu den Gradiertenbasierten Verfahren gehören das Gradientenabstiegsverfahren, das Gradientenabstiegsverfahren mit Nebenbedingungen oder das Quasi-Newton-Verfahren. Sie erfordern lediglich ein kleines Auslenken der Steuerparameter der Anlage aus ihrem Betriebszustand.

Gradienten-basierte Verfahren haben den Vorteil, dass sie für die Umgebung des Betriebszustandes ein sehr präzises Modell des jeweiligen Systems bereit stellen, das einfach und schnell mit einer Auslenkung eines Steuerparameters ermittelt werden kann.

Nicht-Gradienten-basierte Verfahren sind z.B. das Simplex-Nelder-Mead-Verfahren oder das Verfahren der Differentiellen Evolution. Sie erfordern ggf. eine Messung der Zielparameter weit außerhalb des Betriebszustands. Da in der Praxis manche Stellglieder physikalisch limitiert sind (insbesondere die Gasverdichter, die entweder hohe Drücke bei geringen Durchsätzen oder hohe Durchsätze bei geringen Drücken bereitstellen können), kann es bei solchen Verfahren vorkommen, dass es nicht mehr möglich ist, die Anlage unter Einhaltung der Nebenbedingungen stark auszulenken und die Zielfunktion zu bestimmen. Eine Übersicht verschiedener Optimierungsmethoden ist z.B. in dem Lehrbuch Optimierung von Florian Jarre und Josef Stoer (DOI 10.1007/978-3-642-18785-8) gegeben.

Eine Biogasaufbereitungsanlage kann manchmal in einem Teillast-Betrieb betrieben werden. Dies hat vor allem zwei Gründe: es steht nicht genügend Rohbiogas für einen dauerhaften Betrieb bei höherer Auslastung zur Verfügung oder der Netzbetreiber muss die EinspeiseAnlage herunterregeln, da das Gasnetz gefüllt ist. Anders als bei Strom-Netzen ist ein Rückströmen des Biomethans aus einem regionalen Niederdruck-Gasnetz in ein überregionales Hochdruck-Verteilernetz nicht ohne weiteres möglich. Es kommt daher in warmen Sommernächten häufiger zu Einspeisedrosselungen bei Aufbereitungsanlagen an kleinen regionalen Gasnetzen.

Bei einer Drosselung verändert sich der Qualitätsparameter Soll-Produktgasstroms. Nach dem Stand der Technik werden die Steuerparameter (Unabhängigen Variablen) so eingestellt, dass die Nebenbedingungen (Einhaltung der Qualitätsparameter Produktgasstrom, Produktgasqualität und Schwachgasqualität) wieder hergestellt werden. Auf eine energetische Optimierung wird jedoch im Allgemeinen verzichtet. Wenn überhaupt, werden einige Verbesserungsschritte manuell durchgeführt (Experten-Regler). Insbesondre, wenn die Aufbereitungsanlage nicht im stationären Dauerbetrieb, sondern flexibel betrieben werden soll oder muss, ist die energetische Optimierung von Vorteil. Bei Blockheizkraftwerken hat sich bei vielen Biogasanlagen bereits der flexible Betrieb gegenüber dem stationären durchgesetzt. Nach einer Änderung der Last der Biogasaufbereitungsanlage wird vorzugsweise das erfindungsgemäße Verfahren zur Optimierung derselben ein jedes Mal ausgeführt.

Eine Biogasanlage im Sinne der vorliegenden Erfindung ist auch eine Power-to-Gas-Anlage. Bei einer solchen Anlage kann auch das erfindungsgemäße Optimierungsverfahren verwendet werden. Wenn auf einer Biogasanlage ein Power-to-Gas-Modul installiert ist, kann Wasserstoff erzeugt werden, der biologisch oder katalytisch in Gegenwart von Kohlendioxid aus dem Biogas zu Methan umgewandelt wird. Das Power-to-Gas-Modul läuft nur, wenn der Strompreis gerade günstig ist (z.B. an einem sonnigen und windigen Wochenende). Wenn das Modul läuft, bleibt der Rohbiogasstrom konstant, der Methangehalt des Rohbiogases steigt jedoch langsam an. Damit ändert sich ein wesentlicher Parameter des Rohbiogases, was weitreichende Anpassungen im Aufbereitungsprozess erfordert. Durch eine erfindungsgemäße, adaptive Regelung des Aufbereitungsprozesses können sich die Systemparameter so anpassen, dass die Nebenbedingungen eingehalten werden und gleichzeitig die Anlage den minimalen Energiebedarf aufweist.

In einer besonderen Ausführungsform der Erfindung werden die Optimierungsschritte so ausgeführt, dass die Anlage an die Qualitätsparameter herangeführt wird. Hierzu wird die Differenzen zwischen den Soll und Ist-Werten der Qualitätsparameter gemessen und bei jedem Schritt verkleinert. Diese Ausführungsform erzeugt ein rückstellendes Element, wenn bei einem Optimierungsschritt leicht von der Einhaltung einer Nebenbedingung abgewichen wird.

Bei einer weiteren besonderen Ausführungsform werden zunächst alle verfügbaren Steuerparameter ausgelenkt und in den nachfolgenden Schritten nur eine kleinere Auswahl der verfügbaren Steuerparameter. Auf diese Weise wird die Annäherung an das Optimum beschleunigt. Nach einer vorher definierten Anzahl an Schritten mit einer reduzierten Anzahl Steuerparametern oder nachdem ein Schritt keine Annäherung an der Optimum mehr ergibt werden wieder alle verfügbaren Steuerparameter ausgelenkt und erneut überprüft, ob alle Steuerparameter wesentlich zu einer Veränderung der Zielparameter, insbesondere zu einer Optimierung der Leistung oder des Durchsatzes, beitragen.

Das Auslenken des Systems muss nicht auf einen Steuerparameter zurzeit beschränkt sein. Man kann beispielsweise mehrere Steuerparameter mittels einer Hauptkomponentenanalyse bündeln und neue Steuerparameter erstellen, die Linearkombinationen oder auch nichtlineare Kombinationen der Steuerparameter sind. Auf diese Weise nimmt die Anzahl der freien unabhängigen Variablen ab und die Iteration kommt schneller zum Ziel. Ggf. muss nach einigen Schritten die Hauptkomponentenanalyse neu ausgeführt werden und die Hauptkomponenten neu berechnet werden.

In einer weiteren bevorzugten Ausführungsform wird als Gasaufbereitungsverfahren ein Gaspermeationsverfahren verwendet und ein Teil der Membranen mit automatischen Armaturen bzw. mittels von einer Steuereinrichtung einstellbaren Ventilen versehen, so dass die Module einzelnen Stufen zugeordnet werden können. Die erfindungsgemäße Regelung kann dann auch die Membranfläche im laufenden Betrieb ändern, um somit flexibel auf Änderungen von außen zu reagieren.

Das Verfahren kann mit herkömmlichen Reglern kombiniert werden. Dies bietet sich insbesondere dann an, wenn der Regler schnell arbeiten kann und die Stellgröße keine hohe Querempfindlichekeit bei anderen Qualitätsparametern verursacht. Der Gasstrom durch die Anlage kann beispielsweise mit einem einfachen PID-Regler eingestellt werden. Dann entfällt eine Nebenbedingung (die Einhaltung eines Gasstroms) und eine unabhängige Variable (die Drehzahl eines Gebläses). Das Optimierungs-Problem wird somit deutlich reduziert.

Die Erfindung wird anhand einer Zeichnung beispielhaft erläutert. Es zeigen dabei:

Fig. 1    das dreistufige Membranverfahren in einem Verfahrensablaufschema;

Fig. 2    eine Tabelle mit dem Verlauf der Betriebsparameter bei der energetischen Optimierung aus Beispiel 1;

Fig. 3    eine graphische Übersicht des Optimierungslaufs aus Figur 2

Fig. 4    eine Tabelle mit dem Verlauf der Betriebsparameter bei der Optimierung des Durchsatzes aus Beispiel 3

Fig. 5    das Verfahrensablaufschema der Druckwasserwäsche aus Beispiel 4

**Erstes Ausführungsbeispiel**

Als Optimierungsverfahren wird das Gradientenabstiegsverfahren (GAV) gewählt, welches um die Einhaltung der Nebenbedingung erweitert ist.

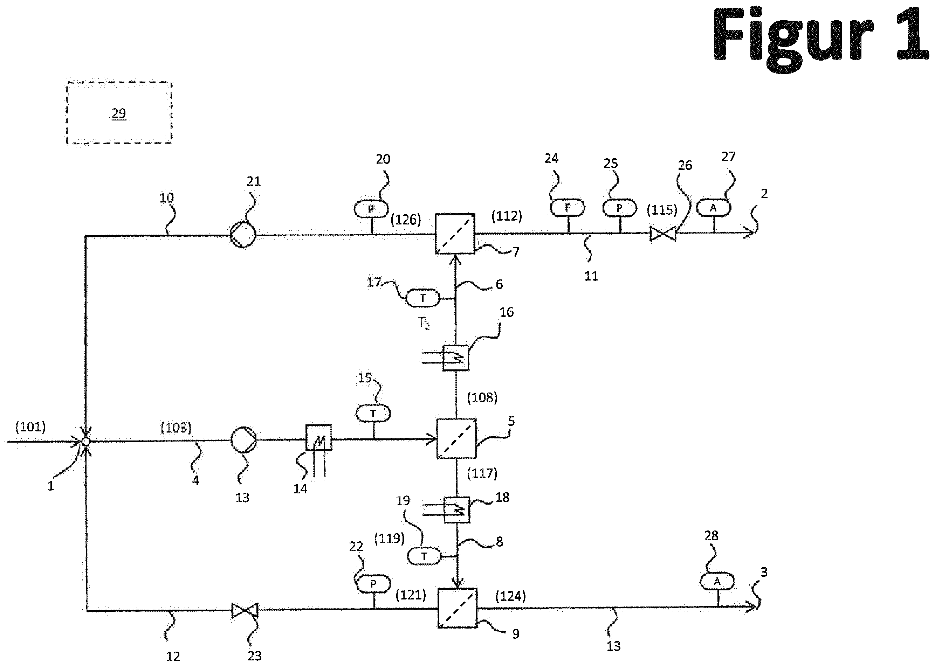

Die Erfindung wird nachfolgend beispielhaft anhand einer Aufbereitungsanlage, welche als Membranfiltereinrichtung mit drei Stufen (Figur 1) ausgebildet ist, erläutert. Die Erfindung kann grundsätzlich auf beliebige Teile von Biogasanlagen und anderen Prozessanalgen, deren Betrieb von einer Vielzahl Parameter abhängt, angewandt werden.

Die dreistufige Membranfiltereinrichtung weist eine Vakuumpumpe 128 auf (Figur 1).

Die Membranfiltereinrichtung weist einen Einlass 1 für getrocknetes Rohbiogas 101 einen ersten Auslass 2 für ein Produktgas, das überwiegend aus Methan besteht und einen zweiten Auslass 3 für ein Schwachgas, das nur einen geringen Methangehalt aufweist, auf. Vom Einlass 1 führt eine Leitung 4 zu einer ersten Membranstufe 5. Die Membranstufe 5 trennt das zugeführte Gas in ein Permeat und in ein Retentat. Die erste Membranstufe besitzt einen Ausgang für das Retentat, der über eine Leitung 6 in einer zweiten Membranstufe 7 verbunden ist. Der Ausgang der ersten Membranstufe 5 für das Permeat ist mit einer Leitung 8 mit einer dritten Membranstufe 9 verbunden.

Die zweite Membranstufe 7 trennt wiederum das zugeführte Gas in ein Permeat und ein Retentat, wobei der Ausgang der zweiten Membranstufe 7 für das Permeat mit einer Leitung 10 verbunden ist, die zurück zum Einlass 1 führt. Der Ausgang der zweiten Membranstufe 7 für das Retentat ist mit einer Leitung 11 verbunden, die zum ersten Auslass 2 für das Produktgas führt.

Die dritte Membranstufe 9 trennt wiederum das zugeführte Gas in ein Permeat und in ein Retentat. Der Auslass der dritten Membranstufe 9 für das Retentat ist mit einer Leitung 12 mit dem Einlass 1 verbunden. Der Auslass der dritten Membranstufe 9 für das Permeat ist mit einer Leitung 13 mit dem zweiten Auslass 3 für das Schwachgas verbunden.

Diese Membranfiltereinrichtung weist somit zwei Kreisläufe (erster Kreislauf: Leitung 4, 6, 10; zweiter Kreislauf: Leitung 4, 8, 12) auf, in welchen ein Teil des aufzubereitenden Gases rezirkuliert werden kann.

In der Leitung 4 befinden sich ein Kompressor 13, ein Wärmetauscher 14 und ein Thermometer 15. In den Leitungen 6 und 8 ist jeweils ein Wärmetauscher 16, 18 bzw. ein Thermometer 17, 19 angeordnet.

In der Leitung 10 befindet sich ein Manometer 20 zum Messen des Druckes des Permeates der zweiten Membranstufe 7. Eine Vakuumpumpe 21 ist zwischen dem Manometer 20 und dem Einlass 1 in der Leitung 10 angeordnet, um das Permeat der zweiten Membranstufe 7 zum Einlass zu pumpen.

In der Leitung 12 befindet sich ein Manometer 22 zum Messen des Retentatdruckes, der dritten Membranstufe 9. Im Bereich zwischen dem Manometer 22 und dem Einlass 1 ist in der Leitung 12 ein Drosselventil 23 angeordnet, mit welchem der Retentatdruck der dritten Membranstufe 9 einstellbar ist.

Die Leitung 11, die vom Ausgang für das Retentat der zweiten Membranstufe 7 zum ersten Auslass 2 für das Produktgas führt, weist aufeinanderfolgend ein Gasstrommessgerät 24, ein Manometer 25, ein Drosselventil 26 und ein Messgerät 27 zum Messen der Gaszusammensetzung, insbesondere zum Messen der Konzentration von $CO_2$, auf.

Dieses Messgerät 27 zum Bestimmen der Gaszusammensetzung kann ein Gassensor sein, der eine bestimmte oder mehrere bestimmte Komponenten der Gaszusammensetzung kontinuierlich misst, oder er kann ein Gaschromatograph sein, wobei der Gaschromatograph unabhängig von der Membranfiltereinrichtung ausgebildet ist und in der Membranfiltereinrichtung lediglich eine Probenentnahmestelle angeordnet ist. In der Leitung 13, welche zum zweiten Auslass 3 für das Schwachgas führt, ist ein weiteres Messgerät 28 zum Messen der Gaszusammensetzung, insbesondere zum Messen der Konzentration von $CH_4$, bzw. eine entsprechende Probenentnahmestelle angeordnet.

Ein entschwefeltes und getrocknetes Rohbiogas 101 wird mit zwei $CO_2$-reichen Rezirkulatströmen 121, 126 im Bereich des Einlasses 1 vermischt und mit dem Kompressor 13 auf einen hohen Druck von etwa 12 bis 18 bar gebracht. Die Temperatur dieses Gasgemisch, das auch als Feed-Gas 103 bezeichnet wird, wird mit dem Wärmetauscher 14 auf einen bestimmten Wert eingestellt und mit dem Thermometer 15 gemessen. Das verdichtete Feed-Gas wird auf die erste Membranstufe 5 gegeben. Die Membranstufe 5 besteht aus einem oder mehreren parallel angeordneten Membranmodulen, die bevorzugt im Gegenstrom betrieben werden. Das Retentat 108 der ersten Stufe 5 strömt bei fast unverändertem Druck zur zweiten Membranstufe 7. Bevor es in die zweite Membranstufe 7 eintritt wird erneut die Temperatur mit dem Wärmetauscher 16 eingestellt und mit dem Thermometer 17 gemessen. Der Methangehalt des Retentats 108 der ersten Stufe 5 beträgt ca. 80%. Die zweite Stufe 7 besteht aus einem oder mehreren parallel angeordneten Membranmodulen, die bevorzugt im Gegenstrom betrieben werden.

In der zweiten Stufe 7 wird das Gas weiter von $CO_2$ befreit, als Retentat entsteht das Produktgas 112 mit einem Methangehalt von über 95% und einem $CO_2$-Gehalten von unter 2%. Die Gasqualität des Produktgases wird mit dem Messgerät 27 erfasst. Der Massenstrom des Produktgases wird mit dem Gasflussmessgerät 24 gemessen. Der Retentatdruck des zweiten Stufe wird mit dem Manometer 25 gemessen und mit dem Drosselventil 26 eingestellt. Das Produktgas kann einer Einspeiseanlage übergeben werden, wo es ggf. temperiert, weiter gedrosselt oder verdichtet sowie odoriert wird und schließlich ins Netz oder in einen Speicher eingespeist wird. Die entsprechende Einspeiseinrichtung ist in Figur 1 nicht dargestellt.

Das Permeat 126 der zweiten Stufe 7 wird wieder zum Einlass 1 über die Leitung 10 befördert, an der ein statischer Mischer angeordnet ist, um es mit dem Rohbiogas 101 zu mischen. Mit der Vakuumpumpe 21 kann der gemessene Permeatdruck weiter abgesenkt werden. Speziell bei hohen Methangehalten kann mit einem hohen Druckverhältnis eine sehr hohe Retentatreinheit erzeugt werden.

Das Permeat 117 der ersten Stufe 5 wird auf die dritte Stufe 9 geleitet. Die dritte Stufe 9 besteht aus einem oder mehreren parallel angeordneten Membranmodulen, die bevorzugt im Gegenstrom betrieben werden. Zuvor wird die Temperatur mit dem Wärmetauscher 18 auf einen definierten Wert eingestellt und mit dem Thermometer 19 überwacht. Das Permeat der dritten Stufe 9 ist der Schwachgasstrom 124. Wird der Schwachgasstrom 124 in die Umgebung abgeleitet, ist der Permeatdruck der dritten Stufe 9 der Druckverlust der Abgasstrecke. Häufig wird das Schwachgas 124 mit Luft vermischt und über einen Kamin in die Umgebung geblasen. Die Vermischung mit Luft ist notwendig, um ein Absinken des Schwachgases auf den Boden in der Nähe der Aufbereitungsanlage zu verhindern.

Es kann notwendig sein, dass Schwachgas 124 noch nachzubehandeln, um Methanemissionen zu minimieren. Hierzu kann ein Mindest-Methangehalt im Schwachgas 124 notwendig sein. Die Zusammensetzung des Schwachgases wird mit der Messeinrichtung 28 überwacht. Das Retentat 121 der dritten Stufe 9 enthält noch größere Methanmengen und wird mit dem Rohbiogas 101 im statischen Mischer vermischt und erneut komprimiert.

Mit dem Drosselventil 23 wird der gemessene Retentatdruck der dritten Stufe 9 eingestellt. Er entspricht ungefähr dem Permeatdruck der ersten Stufe 5. In der Praxis werden die Anlagen mit ca. 2 bis 4 bar Retentatdruck der dritten Stufe 9 betrieben. Dieser Parameter hat einen extremen Einfluss auf fast alle Nebenbedingungen und auf die Leistungsaufnahme.

Die Temperatur hat einen wesentlichen Einfluss auf das Trennergebnis der Membranen. Bei höheren Temperaturen verbessern sich die Permeabilitäten von Methan und $CO_2$, jedoch steigt die Permeabilität des Methans schneller an als die des $CO_2$. Als Folge sinkt das Verhältnis der Permeabilitäten mit steigender Temperatur und die Selektivität der $CO_2/CH_4$-Trennung sinkt. Es bietet sich daher an, vor jeder Membranstufe 5, 7, 9 einen Wärmetauscher 14, 16, 18 zu installieren und die Temperaturen jeweils auf einen Sollwert einzustellen. Aus Kostengründen können die Wärmetauscher 16, 188 vor der zweiten 7 und dritten 7 Stufe eingespart werden. Um den Prozess zumindest ein wenig zu beeinflussen, kann der Raum, in dem die Membrananlage steht, geheizt oder gekühlt werden.

Die Leistungsaufnahme der Anlage setzt sich im Wesentlichen aus der Leistung des Kompressors 13 und der Leistung der Vakuumpumpe 21 zusammen. Die Leistung der Kühlwasserpumpen kann vernachlässigt werden. Als Kompressor 14 kann ein- oder mehrstufige Kolbenkompressoren oder Schraubenkompressoren zum Einsatz kommen. Schraubenkompressoren können als annähernd isotherm, Kolbenkompressoren als annähend isentrop betrachtet werden. Die Vakuumpumpe 21 ist meist ein Zentrifugal-Gebläse und arbeitet annähernd isentrop. Die Leistungsaufnahme des Kompressors 13 und der Vakuumpumpe 21 hängt von dem Druckverhältnis und von dem Volumenstrom ab.

Rüstet man einen Teil der Membranmodule mit automatischen Ventilen aus, kann man im laufenden Betrieb einzelne Membranmodule unterschiedlichen Stufen zuordnen.

Eine Steuereinheit 29 regelt die Anlage so, dass die Qualitätsparameter eingehalten werden und die Anlage bei einem minimalen Energiebedarf oder einem maximalen Durchsatz betrieben wird.

In der flexibelsten Ausführung hat der dreistufige Prozess acht unabhängige Variablen, welche Steuerparameter bilden:

- Retentatdruck zweite Stufe 7 (Stellgröße Drosselventil 26)
- Permeatdruck zweite Stufe 7 (Stellgröße Drehzahl Vakuumpumpe 21)
- Retentatdruck dritte Stufe 9 (Stellgröße Drosselventil 23)
- Temperatur Eingang erste Stufe 5
- Temperatur Eingang zweite Stufe 7
- Temperatur Eingang dritte Stufe 9
- Anzahl Membranen zweite Stufe 7
- Anzahl Membranen dritte Stufe 9

Die Anzahl der Membranen in der ersten Stufe 5 ergibt sich aus der Gesamtanzahl und der Anzahl der Membranen in der zweiten und dritten Stufe 7, 9.

Die Gesamtanzahl an Membranen wird vorgegeben. Es können einzelne Membranen zwischen den Stufen hin- und hergeschoben werden. In der vorliegenden Ausführungsform regelt ein zusätzlicher Regler, z.B. ein PID-Regler, den Produktgasstrom auf einen Sollwert. Die Stellgröße ist die Kompressordrehzahl.

Man kann sich auch leicht andere Variablensätze überlegen, z.B. die Anzahl der Module in der ersten Stufe als zusätzlichen Freiheitsgrad wählen, oder mit einem zusätzlichen Gebläse ein Vakuum auf der Permeatseite der dritten Stufe erzeugen. Der grundlegende Lösungsalgorithmus bleibt jedoch im Wesentlichen derselbe.

Zunächst werden für alle Steuerparameter Ober- und Untergrenzen festgelegt. Es wird ein Zustandsvektor definiert, der die dimensionslosen unabhängigen Variablen enthält. Er beschreibt einen Punkt im 8-dimensionalen Zustandsraum. Der Zustandsvektor der in Fig. 1 gezeigten Anlage x besteht aus den 8 unabhängigen dimensionslosen Steuerparameter und ist wie folgt definiert:

$$x = \begin{pmatrix} \dfrac{p_{R2} - p_{R2,min}}{p_{R2,max} - p_{R2,min}} \\ \dfrac{p_{P2} - p_{P,min}}{p_{P,max} - p_{P,min}} \\ \dfrac{p_{R3} - p_{R3,min}}{p_{R3,max} - p_{R3,min}} \\ \dfrac{T_1 - T_{min}}{T_{max} - T_{min}} \\ \dfrac{T_2 - T_{min}}{T_{max} - T_{min}} \\ \dfrac{T_3 - T_{min}}{T_{max} - T_{min}} \\ n_2 \\ n_3 \end{pmatrix}$$

mit

| | | |
|---|---|---|
| $p_{R2}$ | [bar] | Retentatdruck der zweiten Stufe |
| $p_{R2,min}$ | [bar] | minimaler Retentatdruck der zweiten Stufe |
| $p_{R2,max}$ | [bar] | maximaler Retentatdruck der zweiten Stufe |
| $p_{P2}$ | [bar] | Permeatdruck der zweiten Stufe |
| $p_{P2,min}$ | [bar] | minimaler Permeatdruck der zweiten Stufe |
| $p_{P2,max}$ | [bar] | maximaler Permeatdruck der zweiten Stufe |
| $p_{R3}$ | [bar] | Retentatdruck der dritten Stufe |
| $p_{R3,min}$ | [bar] | minimaler Retentatdruck der dritten Stufe |
| $p_{R3,max}$ | [bar] | maximaler Retentatdruck der dritten Stufe |
| $T_1$ | [°C] | Eintrittstemperatur der ersten Stufe |

(fortgesetzt)

| | | |
|---|---|---|
| $T_2$ | [°C] | Eintrittstemperatur der zweiten Stufe |
| $T_3$ | [°C] | Eintrittstemperatur der dritten Stufe |
| $T_{min}$ | [°C] | minimale Temperatur |
| $T_{max}$ | [°C] | Maximale Temerpatur |
| $n_2$ | [-] | Anzahl der Membranen der zweiten Stufe |
| $n_2$ | [-] | Anzahl der Membranen der dritten Stufe |

Die Anzahl der Membranen auf der ersten Stufe ergibt sich aus

$$n_1 = n_{ges} - n_2 - n_3$$

Die minimalen und maximalen Drücke und Temperaturen ergeben sich aus dem maximalen Betriebsdruck der Verdichter und Vakuumpumpe sowie der maximalen Betriebstemperatur der Membranmodule.

Im vorliegenden Ausführungsbeispiel soll die in Figur 1 gezeigte Membranfilteranlage bezüglich Ihrer Leistung P so optimiert und insbesondere minimiert werden, dass die Produkt- und Schwachgasreinheit konstant bleibt. Die Leistung P stellt somit den Zielparameter dar. Die Qualitätsparameter werden mit folgendem Vektor **y** beschrieben.

$$\boldsymbol{y} = \begin{pmatrix} x_{CO_2,PG} \\ x_{CH_4,SG} \end{pmatrix}$$

Mit

| | | |
|---|---|---|
| $P$ | [kW] | Leistungsbedarf der Anlage |
| $x_{CO_2,PG}$ | [mol/mol] | $CO_2$-Gehalt Produktgas |
| $x_{CH_4,SG}$ | [mol/mol] | $CH_4$-Gehalt Schwachgas |

Die Optimierung soll somit unter der Nebenbedingung erfolgen, dass der Qualitätsparameter **y** konstant bleibt oder sich zumindest nicht verschlechtert. Es liegen somit zwei Nebenbedingungen zur Erhaltung des $CO_2$-Gehaltes im Produktgas und zur Erhaltung des $CH_4$-Gehaltes vor. Die Einhaltung der Nebenbedingungen kann sichergestellt werden, wenn pro Nebenbedingung eine unabhängige Variable bzw. ein Steuerparameter zu einer abhängigen Variablen bzw. abhängigen Steuerparameter, gemacht wird. Im vorliegenden Ausführungsbeispiel wird der Permeatdruck der zweiten Stufe 7 zur Regelung des $CO_2$-Gehaltes und der Retentatdruck der dritten Stufe 9 zur Regelung des Methangehaltes zu einer abhängigen Variablen $v_i$ gemacht.

Basierend auf dem Anfangspunkt $x_0$, $y_0$ wird die Anlage nacheinander jeweils um $\pm\delta x_i$ ausgelenkt und die entsprechenden Qualitätsparameter $y_{i\pm}$ notiert. Bei der Anzahl der Membranen werden bei dem positiv-Schritt an der zweiten bzw. dritten Stufe 7, 9 ein Modul hinzugefügt und in der ersten Stufe 5 ein Modul entfernt. Beim negativ-Schritt wird an der zweiten bzw. dritten Stufe 7, 9 ein Modul entfernt und der ersten Stufe 5 zugeführt.

Durch das Auslenken der Anlage sollen partielle Ableitungen für die Qualitätsparameter $y_i$, welche die Nebenbedingungen definieren, und für den Zielparameter P gebildet werden. Beim Auslenken werden somit an einem bestimmten Zustand **x** der Anlage die unabhängigen Steuerparameter, jeweils einzeln um einen vorbestimmten Betrag ausgelenkt und dann wieder auf den Wert des Zustandes eingestellt. Das Auslenken erfolgt vorzugsweise so, dass der jeweilige unabhängige Steuerparameter $x_i$ um den vorbestimmten Betrag $\delta x_i$ zunächst verringert und danach vergrößert wird und dann wieder auf den jeweiligen Wert des Zustandes **x** eingestellt wird. Das Auslenken kann natürlich auch so ausgeführt werden, dass der unabhängige Steuerparameter zunächst um den vorbestimmten Wert vergrößert und dann verkleinert wird. Das Auslenken erfolgt vorzugsweise so, dass jeweils nur ein einzelner unabhängiger Steuerparameter ausgelenkt wird. Im ausgelenkten Zustand werden dann die Werte für den Zielparameter (im vorliegenden Ausführungsbeispiel die Leistung P) und die für die Qualitätsparameter $y_i$ gemessen.

Da das Auslenken der Anlage den aufwändigsten Schritt darstellt, kann man zur groben Bestimmung der partiellen Ableitungen einen Steuerparameter auch nur einseitig auslenken. Man kommt dann schneller zum Optimum auf Kosten der Genauigkeit. Die einseitige Auslenkung bietet sich also besonders zu Beginn des Iterationsverfahrens an.

Die partiellen Ableitungen für die Qualitätsparameter $y_i$ werden definiert als

$$\frac{\partial y_j}{\partial x_i} = \frac{y_{j+} - y_{j-}}{2\delta x_i}.$$

Hat eine der Koordinaten ihr Minimum bzw. Maximum erreicht, kann der Schritt zur Bestimmung der Steigung nur in eine Richtung durchgeführt werden:

$$\left.\frac{\partial y_j}{\partial x_i}\right|_{x_i = x_{i,max}} = \frac{y_j - y_{j-}}{\delta x_i}$$

$$\left.\frac{\partial y_j}{\partial x_i}\right|_{x = x_{i,min}} = \frac{y_{j+} - y_j}{\delta x_i}$$

Es werden auch die partiellen Ableitungen für die Variable des zu optimierenden Wertes, also des Zielparameters, der im vorliegenden Ausführungsbeispiel die Leistung P ist, $\frac{\partial P}{\partial x}$ bestimmt.

Die Änderung des Zustandsvektors muss so ausgeführt werden, dass die Nebenbedingungen erfüllt werden und somit die Qualitätsparameter konstant bleiben. Daher gilt:

$$y(x) = y(x + \Delta x)$$

$$0 = y(x + \Delta x) - y(x)$$

$\Delta x$ für kleine

$$y(x + \Delta x) = y(x) + J\Delta x$$

also

$J\Delta x = 0$

$J$ ist die Jacobi-Matrix, welche die partiellen Ableitungen der Qualitätsparameter $y_i$, nach den Variablen $x_i$ umfasst:

$$j_{ij} = \frac{\partial y_i}{\partial x_j}$$

Wenn es möglich ist, die Werte der Qualitätsparameter kontinuierlich zu messen, dann können pro kontinuierlich gemessenem Qualitätsparameter ein die Steuerparameter mit einem einfachen Regler, z.B. einem PID-Regler, geregelt werden. In den meisten Anlagen, die ein mehrstufiges Gaspermeationsverfahren einsetzen, ist ein zentrales Gasmesssystem installiert, welches von verschiedenen Punkten im Prozess Gas ansaugt und analysiert. Zwischen den Messungen liegen im Allgemeinen 10 bis 20 Minuten, in denen das Messgerät Gas ansaugt, misst und danach die Sensoren mit Luft spült. Ohne den Einbau von speziellen Sensoren, wie z.B. FTIR-Spektrometer am Messort ist es schwierig die Produkt- und Schwachgasqualitäten kontinuierlich zu messen. Werden die abhängigen Variablen nur diskontinuierlich gemessen, dann ist es sinnvoll das im Folgenden beschriebene Verfahren zu verwenden.

Es werden zwei Unterräume U und V des Definitionsbereichs X definiert. U ist der Unterraum der unabhängigen Variablen bzw. der unabhängigen Steuerparameter. V ist der Unterraum der abhängigen Variablen bzw. der abhängigen Steuerparameter. Wir weisen den Permeatdruck der zweiten Stufe und den Retentatdruck der dritten Stufe dem Unterraum V zu und alle anderen Variablen dem Unterraum U.

Grundsätzlich kann man auch andere Kombinationen vorgeben. Es empfiehlt sich jedoch die Dimensionen mit

den betragskleinsten Änderungen $\dfrac{\partial P_{ges}}{\partial x_i}$ dem Raum U zuzuordnen. Dies hat zur Folge, dass große Reduktionen der Zielparameter bei relativ kleiner Änderung der Zustandsvariable $\Delta\boldsymbol{x}$ erfolgt.

Für die zwei Unterräume U, V kann man zwei Jacobi-Matrizen **A, B** aufstellen, wobei die Matrix **A** die partiellen Ableitungen der Qualitätsparameter $y_i$ nach den unabhängigen Steuerparametern $u_i$ und die Matrix **B** die partiellen Ableitungen der Qualitätsparameter $y_i$, nach den abhängigen Steuerparametern $v_i$ umfasst. Es gilt:

$$A = \alpha_{ij} = \frac{\partial y_i}{\partial u_j}, \quad B = \beta_{ij} = \frac{\partial y_i}{\partial v_j}$$

Die Nebenbedingung, dass sich die Qualitätsparameter nicht ändern sollen, können mit den beiden Matrizen **A, B** folgendermaßen dargestellt werden:

$$\boldsymbol{A} \cdot \Delta\boldsymbol{u} + \boldsymbol{B} \cdot \Delta\boldsymbol{v} = 0$$

$$\Delta\boldsymbol{v} = -\boldsymbol{B}^{-1} \cdot \boldsymbol{A} \cdot \Delta\boldsymbol{u}$$

Die Länge des Schritts wird mit $\Delta P_{soll}$ vorgegeben.

$$\Delta P_{soll} = \Delta\boldsymbol{x}^T \cdot \frac{\partial P}{\partial \boldsymbol{x}} = \Delta\boldsymbol{u}^T \cdot \frac{\partial P}{\partial \boldsymbol{u}} + \Delta\boldsymbol{v}^T \cdot \frac{\partial P}{\partial \boldsymbol{v}}$$

Aus dem Strahlensatz folgt:

$$\frac{\Delta u_1}{\Delta u_i} = \frac{\frac{\partial P}{\partial u_1}}{\frac{\partial P}{\partial u_i}} \;\Leftrightarrow\; \Delta u_i = \frac{\frac{\partial P}{\partial u_i}}{\frac{\partial P}{\partial u_1}}\Delta u_1 \;\Leftrightarrow\; \Delta\boldsymbol{u} = \frac{\partial P}{\partial \boldsymbol{u}}\frac{\Delta u_1}{\frac{\partial P}{\partial u_1}}$$

$$\Delta P_{soll} = \frac{\Delta u_1}{\frac{\partial P}{\partial u_1}}\left(\frac{\partial P}{\partial \boldsymbol{u}}\right)^T \cdot \frac{\partial P}{\partial \boldsymbol{u}} + \left(-\boldsymbol{B}^{-1}\cdot\boldsymbol{A}\cdot\frac{\partial P}{\partial \boldsymbol{u}}\frac{\Delta u_1}{\frac{\partial P}{\partial u_1}}\right)^T \cdot \frac{\partial P}{\partial \boldsymbol{v}}$$

Und schließlich

$$\Delta u_1 = \frac{\Delta P_{soll} \cdot \frac{\partial P}{\partial u_1}}{\left(\frac{\partial P}{\partial \boldsymbol{u}}\right)^T \cdot \frac{\partial P}{\partial \boldsymbol{u}} + \left(-\boldsymbol{B}^{-1}\cdot\boldsymbol{A}\cdot\frac{\partial P}{\partial \boldsymbol{u}}\right)^T \cdot \frac{\partial P}{\partial \boldsymbol{v}}}$$

$$\Delta\boldsymbol{u} = \frac{\Delta P_{soll} \cdot \frac{\partial P}{\partial \boldsymbol{u}}}{\left(\frac{\partial P}{\partial \boldsymbol{u}}\right)^T \cdot \frac{\partial P}{\partial \boldsymbol{u}} + \left(-\boldsymbol{B}^{-1}\cdot\boldsymbol{A}\cdot\frac{\partial P}{\partial \boldsymbol{u}}\right)^T \cdot \frac{\partial P}{\partial \boldsymbol{v}}}$$

Mit dieser Formel kann somit für ein vorgegebenes $\Delta P_{soll}$ die entsprechende Zustandsänderung der unabhängigen Steuerparameter $\Delta u$ berechnet werden.

Da die Zustandsänderung der abhängigen Steuerparameter $\Delta\boldsymbol{v}$ von der Zustandsänderung der unabhängigen

Steuerparameter $\Delta u$ abhängt, damit die Nebenbedingen eingehalten werden können, kann mit dem oben angegebenen Gleichungssystem für die Nebenbedingungen mit den Matrizen $A$, $B$ die Zustandsänderung der abhängigen Steuerparameter $\Delta v$ berechnet werden. Aus $\Delta u$ und $\Delta v$ wird $\Delta x$ gebildet. Der neue Punkt im Zustandsraum berechnet sich über:

$$x_{neu} = x_{alt} + \Delta x$$

Es muss noch überprüft werden, ob der Zustandsvektor außerhalb des Definitionsbereichs liegt. Ggf. muss eine Variable auf 0 oder 1 begrenzt werden. Es liegt dann ein Randoptimum vor. Führt im folgenden Iterationsschritt die Änderung aus dem Definitionsbereich heraus, werden die partiellen Ableitungen dieses Steuerparameters zu null gesetzt. Das Optimierungsproblem wird dann um eine Dimension reduziert.

Die Anzahl der Module pro Membranstufe muss auf ganze Zahlen gerundet werden. Es tritt dann eine leichte Abweichung von den Nebenbedingungen auf.

Das Verfahren kann auch so abgewandelt werden, dass nicht bei jeder Iteration die Werte die Werte für die Qualitätsparameter $y_i$ gemessen werden. Das ist dann zweckmäßig, wenn die jeweilige Messung sehr aufwändig ist, wie z.B. bei der Bestimmung der Gaszusammensetzung. Man kann dann über einige, vorzugsweise wenige Iterationsschritte die Qualitätsparameter $y_i$, nicht beobachten, wodurch sie etwas von ihrem jeweiligen Sollwert abweichen können.

Solche Abweichungen können auch durch Rundungsfehler bei permanenter Überwachung der Qualitätsparameter $y_i$, auftreten.

Um die Einhaltung der Nebenbedingungen auch bei solchen Abweichungen zu gewährleisten, kann das Verfahren etwas modifiziert werden.

Hierzu wird die Abweichung $\Delta y$ der Qualitätsparameter $y_i$, definiert:

$$\Delta y = (y_{soll} - y_{ist})$$

Dann soll für einen jeden Iterationsschritt gelten

$$J \Delta x = \alpha \cdot \Delta y$$
,

wobei der Skalierungsfaktor $\alpha$ dazu dient bei einem jeden Iterationsschritt nur einen kleinen Schritt in Richtung Einhaltung der Nebenbedingungen zu machen, während der Leistungsbedarf minimiert wird. Er sollte klein sein, wenn $\Delta y$ groß ist und wird 1, wenn die Nebenbedingungen erreicht werden. Hierdurch erfolgt eine zweite Optimierung, die unabhängig von der Optimierung der Zielfunktion ist, indem die Qualitätsparameter $y_i$, schrittweise in Richtung zu ihren Sollwerten geführt werden.

Und schließlich

$$\Delta v = B^{-1} \cdot (\alpha \cdot \Delta y - A \cdot \Delta u)$$

Die Länge von $\Delta u$ berechnet sich zu:

$$\Delta u = \frac{\partial P}{\partial u} \frac{\Delta P_{soll} - (B^{-1} \cdot \alpha \cdot \Delta y)^T \cdot \frac{\partial P}{\partial v}}{\left(\frac{\partial P}{\partial u}\right)^T \cdot \frac{\partial P}{\partial u} - \left(B^{-1} \cdot A \cdot \frac{\partial P}{\partial u}\right)^T \cdot \frac{\partial P}{\partial v}}$$

An den restlichen Berechnungen ändert sich nichts.

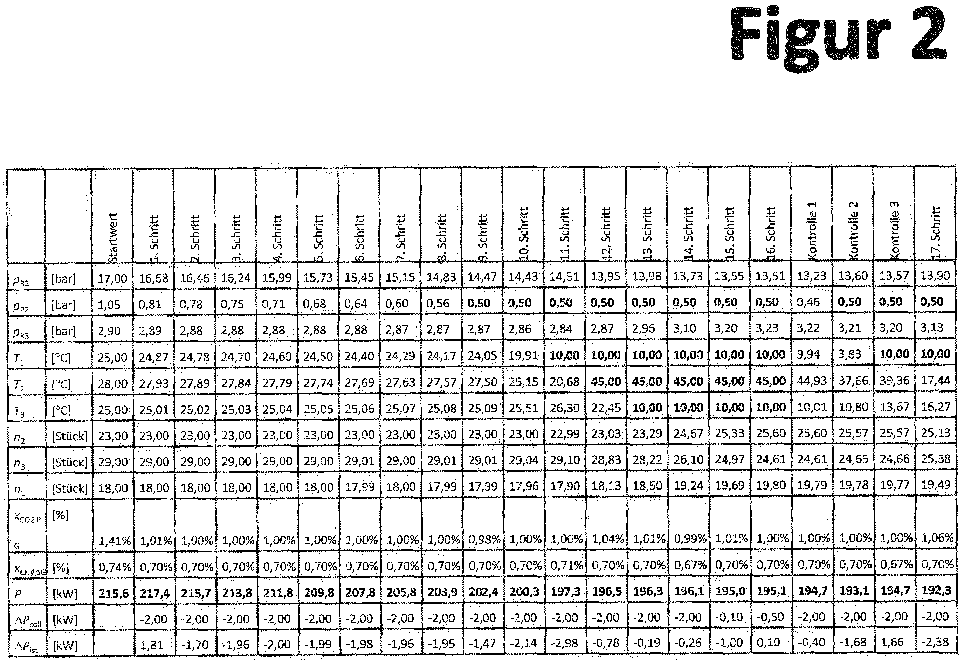

Nachfolgend wird die Anwendung dieses Verfahren zur Optimierung der in Fig. 1 gezeigten Membranfiltereinrichtung anhand eines konkreten Zahlenbeispiels erläutert. In Figur 2 sind die Zahlenwerte der einzelnen Parameter in den einzelnen Optimierungsschritten aufgeführt. Fettgedruckte Parameter liegen auf dem Rand des Definitionsbereiches. Die Sollwerte für $x_{CO2,PG}$ und $x_{CH4,SG}$ sind 1,0% bzw. 0,7%.

Die Membranfiltereinrichtung soll aus 836,5 Nm³/h Rohbiogas mit der Zusammensetzung 53% $CH_4$, 43,2% $CO_2$, 0,3% $N_2$, 0,2% $O_2$ ein Produktgas mit einem $CO_2$-Gehalt von 1% und ein Schwachgas mit einem $CH_4$-Gehalt von 0,7% herstellen. Als Startbedingungen wird eine Membranverschaltung von 18 Membranen in der ersten Stufe, 23 Membranen in der zweiten Stufe und 29 Membranen in der dritten Stufe gewählt. Insgesamt sind 70 Membranmodule verbaut. Die Anzahl der Module wird zunächst nicht gerundet. Die Drücke betragen 17 bar im Retentat der zweiten Stufe, 2,9 bar im Retentat der dritten Stufe und 1,05 bar im Permeat der zweiten und dritten Stufe. Die Temperaturen betragen 25 °C am Eingang der ersten und dritten Stufe sowie 28 °C im Eingang der zweiten Stufe. Das Trennverhalten der Membran wird isotherm und isobar gerechnet. Mit diesen Parametern werden die folgenden Qualitätsparameter gemessen:

$$y = \begin{pmatrix} 1,45\% \\ 0,74\% \end{pmatrix}, \Delta y = \begin{pmatrix} -0,45\% \\ -0,04\% \end{pmatrix}$$

Es herrscht insbesondere bei der Produktgasqualität noch eine deutliche Abweichung vom Sollwert.

Die Anlage weist einen Leistungsbedarf von P = 215,6 kW auf, was 0,258 kWh/Nm$^3$ entspricht. Es werden Ober- und Untergrenzen für die einzelnen Parameter definiert und die Parameter dimensionslos gemacht. Die Temperatur darf zwischen 10 und 45 °C liegen, der Retentatdruck der zweiten Stufe zwischen 7 und 18 bar, der Permeatdruck der zweiten Stufe zwischen 0,5 und 1,1 bar, der Retentatdruck der dritten Stufe zwischen 1 und 5 bar.

Jetzt werden nacheinander die unabhängigen Steuerparameter ausgelenkt und die Veränderung der Leistung und der Qualitätsparameter beobachtet. Die Auslenkungen betragen 5% bei den Drücken und Temperaturen und ein Modul bei den Membranen. Die Änderungen sind des besseren Verständnisses halber dimensionsbehaftet angegeben:

| | $\dfrac{\partial P}{dx}$ | $x_{CO_2,PG}$ | $\dfrac{\partial y_1}{dx}$ | $x_{CH_4,SG}$ | $\dfrac{\partial y_2}{dx}$ |
|---|---|---|---|---|---|
| $p_{R2}$ | 13,39 kW/bar | -0,365 %/bar | | -0,031 %/bar | |
| $p_{P2}$ | -5,70 kW/bar | 2,255 %/bar | | 0,099 %/bar | |
| $p_{R3}$ | -80,89 kW/bar | -0,139 %/bar | | 1,912 %/bar | |
| $T_1$ | 0,79 kW/°C | -0,014 %/°C | | -0,003 %/°C | |
| $T_2$ | 0,27 kW/°C | -0,015 %/°C | | -0,001 %/°C | |
| $T_3$ | -0,05 kW/°C | 0,000% %/°C | | 0,011 %/°C | |
| $n_2$ | -0,55 KW/Modul | -0,068 %/Modul | | 0,007 %/Modul | |
| $n_3$ | -5,52 kW/Modul | 0,014 %/Modul | | 0,131 %/Modul | |

Man erkennt die unterschiedlich starken Abhängigkeiten der Ziel- und Qualitätsparameter von den unterschiedlichen unabhängigen Steuerparametern. Erhöht man beispielsweise den Retentatdruck der zweiten Stufe um ein bar, steigt die Leistungsaufnahme der Anlage um 13,4 kW. Erhöht man den Retentatdruck der dritten Stufe um ein bar, sinkt die Leistungsaufnahme um 80,9 kW, gleichzeitig steigt aber die Methan-Konzentration im Schwachgas um 1,912% an. Höhere Temperaturen führen zur Reduktion fast aller Qualitätsparameter, nur in der dritten Stufe verschlechtern sich Produkt- und Schwachgasqualität leicht mit steigender Temperatur. Fügt man ein Modul von der ersten in die zweite Stufe ein, sinkt der Energiebedarf der Anlage leicht, der CO$_2$-Anteil im Produktgas reduziert sich und der Methananteil im Schwachgas steigt leicht an. Entnimmt man ein Modul aus der ersten Stufe und überführt es in die dritte Stufe sinkt der Energiebedarf deutlich und die Produkt- und Schwachgasqualitäten verschlechtern sich.

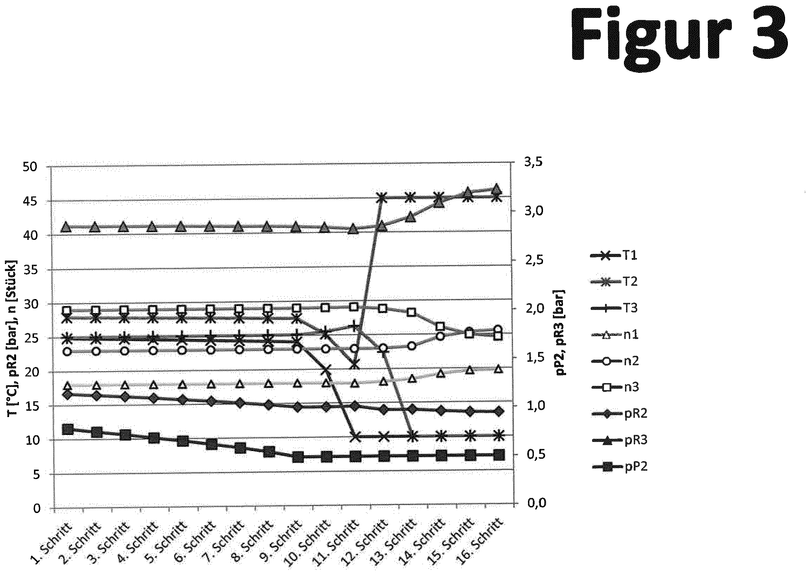

Die Schrittlänge $P_{soll}$ wird zu -2 kW gesetzt, was ca. 1% der Anlagenleistung darstellt. Der Skalierungsfaktor $\alpha$ wird zu 1 gesetzt, da die Qualitätsparameter bereits in guter Nähe zu den Sollwerten liegen. Der Verlauf der Optimierung ist in Figur 2 und 3 aufgeführt.

Für den ersten Schritt berechnet sich die Zustandsänderung zu

$$\Delta x = \begin{pmatrix} -0,32\ bar \\ -0,24\ bar \\ -0,01\ bar \\ -0,13\ °C \\ -0,07\ °C \\ +0,01\ °C \\ +0,000 \\ +0,001 \end{pmatrix}$$

Die Abweichung von den Qualitätsparametern beträgt jetzt:

$$\Delta y = \begin{pmatrix} -0,006\% \\ +0,000\% \end{pmatrix}$$

Bereits im ersten Schritt werden die Qualitätsparameter weitestgehend hergestellt. Allerdings steigt deswegen auch die Leistung um 1,8 kW auf

$P = 217,4\ kW$

In den folgenden 9 Schritten sinken Permeat- und Retentatdruck der zweiten Stufe bis schließlich der Permeatdruck den Rand des Definitionsbereichs bei 0,5 erreicht hat. Bis zu diesem Punkt wird die Leistung auf 202,4 kW gesenkt. Jetzt wird der Druck auf einen konstanten Wert geregelt und der Permeatdruck der zweiten Stufe nicht mehr als unabhängiger Steuerparameter betrachtet. Stattdessen wird jetzt der Retentatdruck der zweiten Stufe als abhängiger Steuerparameter betrachtet und dem Unterraum V zugewiesen. U reduziert sich um eine Dimension.

Im zehnten Schritt setzt ein Absinken der Temperatur am Eingang der ersten Stufe ein, um im 11. Schritt bereits am Rand des Definitionsbereiches (10 °C) anzukommen. Für den folgenden Schritt wird die Temperatur am Eingang der ersten Stufe als konstant angesetzt und die Anzahl der freien Steuerparametererneut um 1 auf 4 reduziert.

Im 12. Schritt steigt die Temperatur am Eingang der zweiten Stufe auf 45 °C, nachdem sie zuvor fast unverändert geblieben war und nur im 11. Schritt um ca. 5 °C abgesunken war. Die Temperatur am Eingang der zweiten Stufe wird jetzt als konstant 45 °C angesehen. Das Optimierungsproblem wird erneut um eine Dimension reduziert.

Im 13. Schritt sinkt die Temperatur am Eingang der dritten Stufe von 22 °C auf 10 °C und wird im Folgenden konstant gehalten. Das Optimierungsproblem hat jetzt nur noch zwei unabhängige Steuerparameter: die Anzahl der Membrane pro Stufe. Der Leistungsbedarf der Anlage ist bis jetzt auf 196,3 kW gesunken.

In den folgenden drei Schritten werden die Anzahl der Module pro Stufe geändert bis sich 19,8 in der ersten Stufe, 25,6 in der zweiten Stufe und 24,6 in der dritten Stufe befinden. Gleichzeitig steigt der Retentatdruck in der dritten Stufe von zuvor 2,95 bar auf 3,2 bar an. Der 16. Schritt führt nicht mehr zu einer Verringerung des Leistungsbedarfs, sondern im Gegenteil zu einem leichten Anstieg. Der 15. Schritt stellt also zunächst das Optimum mit 195,05 kW dar. Jetzt wird nochmal überprüft, ob wirklich in allen Dimensionen $p_{P2}$, $T_1$, $T_2$ und $T_3$ ein Randoptimum vorliegt. Hierzu wird ein Kontrollschritt durchgeführt, bei dem wieder in allen Dimensionen ausgelenkt wird und anschließend das $\Delta x$ für 2 bis 6 unabhängige Variable berechnet wird. Wären alle 6 Variablen wieder unabhängig, würde der Permeatdruck der zweiten Stufe im nächsten Schritt weiter abgesenkt werden und auch die Temperatur am Eingang der ersten Stufe würde auf 9 °C fallen. Der Schritt wäre also unzulässig. In der nächsten Kontrollrechnung wird der Permeatdruck der zweiten Stufe wieder konstant zu 0,5 bar gesetzt und die Reaktion berechnet. In der zweiten Kontrollrechnung würde die Temperatur am Eingang der 1. Stufe auf 3,2 °C fallen. Auch dieser Schritt wäre unzulässig. In der dritten Kontrollrechnung wird die Temperatur am Eingang der ersten Stufe und der Permeatdruck der zweiten Stufe konstant gehalten. Der resultierende Optimierungsschritt führt zu einem Zustandspunkt der im Definitionsbereich liegt. Die Temperatur am Eingang der zweiten Stufe sinkt von 45 °C auf 39 °C, die Temperatur am Eingang der dritten Stufe steigt leicht um 3 °C an. Das Verfahren kann mit vier unabhängigen Variablen weitergehen.

Im 17. Optimierungsschritt fällt die Temperatur am Eingang der zweiten Stufe von 39 °C auf 17 °C und die Temperatur am Eingang der dritten Stufe liegt bei 16 °C. Die Nebenbedingungen werden noch ungefähr eingehalten. Die Ausschläge werden sehr groß.

Im letzten Schritt steigt bei einer Schrittweite von -2 kW die Temperatur am Eingang der zweiten Stufe wieder sprunghaft auf 103 °C an, die Temperatur am Eingang der dritten Stufe fällt auf 4 °C. Selbst wenn die Schrittweite auf 0 kW gesetzt wird, liegt die Temperatur am Eingang der zweiten Stufe bei 53 °C. Auch wenn also keine Verbesserung mehr erzielt wird, würde der nächste Schritt aus dem Definitionsbereich führen. Die Optimierungsroutine wird abgebrochen. Der 17. Schritt hat das Optimum erreicht.

Durch die Optimierung wurde der Leistungsbedarf der Anlage von 215,6 kW auf 192,3 kW um 10,8% abgesenkt. Die Ersparnis beträgt ca. 195.000 kWh/a, was zurzeit ca. 30.000 €/a entspricht.

Zur Erreichung des Optimums sind 17 Schritte notwendig, die ersten neun davon gehen ungefähr in dieselbe Richtung. Das Gradientenverfahren gibt zunächst nur die Richtung der Zustandsänderung vor, in der Rechnung oben wird der Vektor **x** so skaliert, dass sich eine Änderung von $P_{soll}$ ergibt. Statt die Länge des Schrittes vorzugeben, kann man den Schritt einfach immer weiter verlängern, bis sich eine Abweichung von den Qualitätsparametern oder ein Anstieg der Leistungsaufnahme bemerkbar macht. Hierzu werden beim Ändern des Anlagenzustands alle Steuerparameter gleichzeitig entlang des Richtungsvektors Δ**x** ausgelenkt. Hierzu wird das $P_{soll}$ sukzessive erhöht, ohne dass ein erneutes Auslenken der Anlage notwendig ist. Der Verzicht auf das erneute Auslenken führt in der Praxis zu einer nicht unerheblichen Zeitersparnis.

Während der Optimierung werden zunächst die Drücke optimiert, dann die Temperaturen und als letztes die Anzahl Membranmodule pro Stufe. Man kann dieses Wissen ausnutzen, um bei der Optimierung die Variation der mehr oder weniger unveränderlichen unabhängigen Steuerparameter wegzulassen. Man würde also zunächst nur die Drücke variieren, dann die Drücke und Temperaturen und zuletzt die Drücke, Temperaturen und Anzahl Module pro Stufe.

**Erste Abwandlung des ersten Ausführungsbeispiels**

Diese Abwandlung des ersten Ausführungsbeispiels entspricht dem oben erläuterten Ausführungsbeispiel, sofern unten nichts anderes ausgeführt ist. Insbesondere erfolgt die Auslenkung der unabhängigen Steuerparameter genauso wie es oben erläutert ist. Lediglich die Nebenbedingungen werden nicht bei den Berechnungen einbezogen, sondern die Einhaltung der Nebenbedingungen wird mittels Regler realisiert. Die Qualitätsparameter kann man auch mittels Regler, z.B. PID-Regler, regeln. Hierzu ist es von Vorteil, wenn die Regelgröße (= Qualitätsparameter) kontinuierlich gemessen wird. Würde man die Anlage aus Beispiel 1 optimieren, kann man folgende Regler-Kaskade wählen:

| # | Regelgröße | Stellgröße | Stellglied | Sollwert |
|---|---|---|---|---|
| 1 | Produktgasstrom | Kompressordrehzahl | FU Kompressor | Vorgabe Biogasanlage |
| 2 | $CO_2$-Gehalt Produktgas | Permeatdruck 2. Stufe | FU + Bypass Vakuumpumpe, | Betrei bervorgabe |
| 3 | $CH_4$-Gehalt Schwachgas | Retentatdruck 3. Stufe | Drossel Retentat 3. Stufe | Betrei bervorgabe |
| 4 | Druck Eingang 1. Stufe | Öffnung Drossel | Drossel Retentat 2. Stufe | Optimierungsroutine |
| 5 | Temperatur Eingang 1. Stufe | Öffnung Ventil | Regelventil Wärmeübertrager 1 | |
| 6 | Temperatur Eingang 2. Stufe | Öffnung Ventil | Regelventil Wärmeübertrager 2 | |
| 7 | Temperatur Eingang 3. Stufe | Öffnung Ventil | Regelventil Wärmeübertrager 3 | |

Der Zustandsvektor hat nur vier Dimensionen:

$$x = \begin{pmatrix} \dfrac{p_1 - p_{min}}{p_{max} - p_{min}} \\ \dfrac{T_1 - T_{min}}{T_{max} - T_{min}} \\ \dfrac{T_2 - T_{min}}{T_{max} - T_{min}} \\ \dfrac{T_3 - T_{min}}{T_{max} - T_{min}} \end{pmatrix}$$

Die Anzahl der Membranen pro Stufe wird hier als konstant angesehen.

Die partielle Ableitung der Zielfunktion wird bestimmt über:

$$\frac{\partial P}{\partial x_i} = \frac{P_i^+ - P_i^-}{2\delta_i}$$

Die Änderung im Zustandsraum berechnet sich zu

$$\Delta x = \frac{\Delta P_{soll} \cdot \overline{\frac{\partial P}{\partial x}}}{\left(\frac{\partial P}{\partial x}\right)^T \frac{\partial P}{\partial x}}$$

Läuft ein Steuerparameter aus dem Definitionsbereich, wird er mit einem Regler, z.B. einem PID-Regler, konstant gehalten. Steuerparameter, die auf dem Rand des Definitionsbereiches liegen, können beim nächsten Schritt nur in eine Richtung ausgelenkt werden. Liegt der nächste berechnete Punkt wieder im Inneren des Definitionsbereiches, wird der Schritt ausgeführt. Ansonsten wird die partielle Ableitung $\frac{\partial P}{\partial x_i}$ zu null gesetzt und der Schritt ausgeführt.

**Zweite Abwandlung des ersten Ausführungsbeispiels**

Statt die Energieeffizienz zu optimieren, kann man mit dem erfindungsgemäßen Verfahren auch den Durchsatz der Anlage maximieren. Das limitierende Element der Aufbereitungsanlage ist meist der Kompressor. Er ist in seiner Leistung begrenzt. Eine hohe Kompressorleistung kann mit einem geringen Druck und einem hohen Durchsatz oder bei einem hohen Druck und geringerem Durchsatz erreicht werden.

Man wählt als zusätzlichen unabhängigen Steuerparameter den Inputstrom. Als zusätzlichen Qualitätsparameter wählt man die Kompressorleistung, die sich aus dem Retentatdruck der zweiten Stufe und den Volumenstrom durch den Kompressor ergibt.

Die unabhängigen Steuerparameter sind:

- Rohbiogasstrom in die Anlage (Stellgröße Drehzahl Kompressor)
- Retentatdruck zweite Stufe (Stellgröße Drosselventil)
- Permeatdruck zweite Stufe (Stellgröße Drehzahl Vakuumpumpe)
- Retentatdruck dritte Stufe (Stellgröße Drosselventil)
- Temperatur Eingang erste Stufe
- Temperatur Eingang zweite Stufe
- Temperatur Eingang dritte Stufe
- Anzahl Membranen zweite Stufe
- Anzahl Membranen dritte Stufe

Die Qualitätsparameter sind

- $CO_2$-Gehalt Produktgas
- $CH_4$-Gehalt Schwachgas
- Kompressorleistung

Der Zielparameter ist

- Produktgasstrom

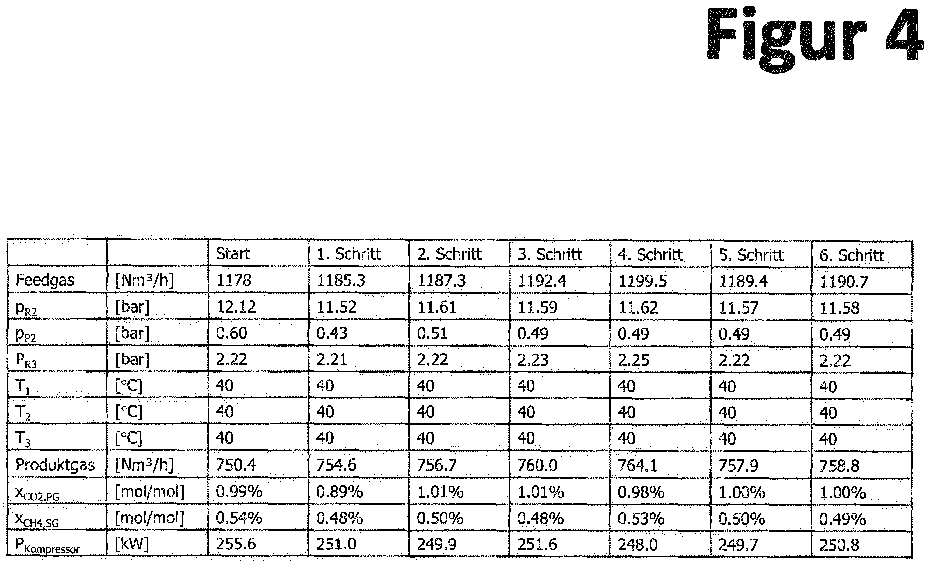

Der Produktgasstrom wird maximiert. Die Figur 4 zeigt die Optimierung. Als Startwerte dienen die Ergebnisse eines vereinfachten mechanistischen Modells. Die Anzahl der Membranen pro Stufe wird konstant gehalten; sie beträgt 38/42/40. Das Rohbiogas besteht aus 63% CH4, 36,7% CO2, 0,2% N2, 0,1% 02. Ab dem 4. Schritt wird der Permeatdruck der zweiten Stufe konstant gehalten. Die Temperaturen werden durch den Optimierungsalgorithmus nicht mehr verändert. Basierend auf zuvor gefundenen Optimum kann der Produktgasstrom um ca. 8,8 Nm³/h erhöht werden, gleichzeitig wird der Leistungsbedarf des Kompressors um 4,8 kW gesenkt. Die Kostenersparnis aufgrund der Leistungsreduktion beträgt rund 6000 €/a, der Mehrerlös durch die maximierte Anlagenkapazität beträgt ca. 46.500 €/a (Stromkosten 15 ct/kWh, Biomethanerlös 6 ct/kWh, 8400 h/a)

**Zweites Ausführungsbeispiel**

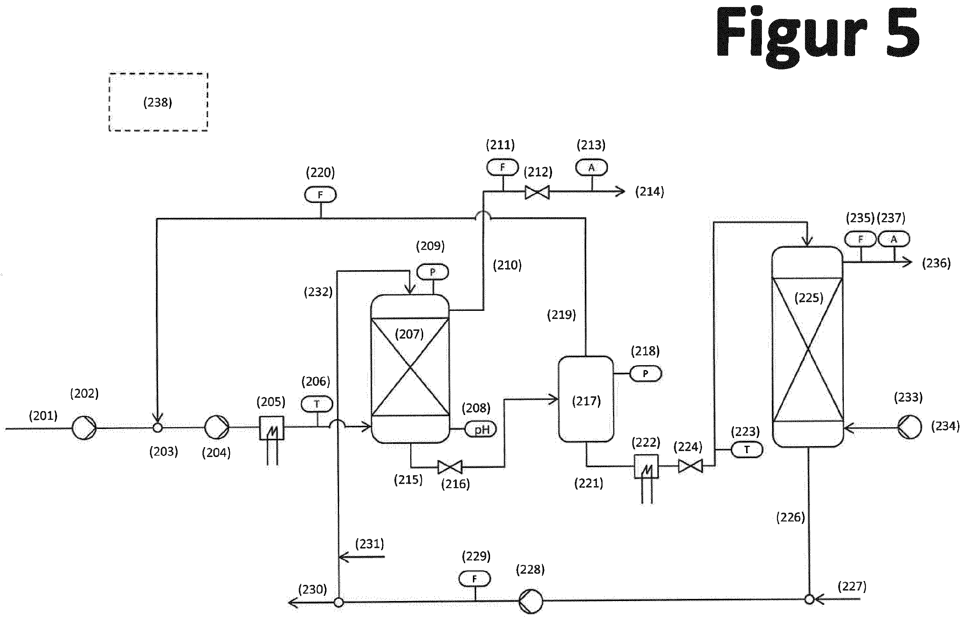

Das Biogas wird mit einer Druckwasserwäsche zu Methan aufgereinigt (Figur 5). Das Rohbiogas 201 wird zunächst mit einem Vorverdichter 202 auf einen Flash-Druck 218 verdichtet und gekühlt und dann mit dem Rezirkulationsgas 219 aus dem Flash-Kessel 217 vermischt 203. Das Gasgemisch wird mit dem Hauptverdichter 204 auf einen vorbestimmten Kolonnendruck 209 einer Absorbtionskolonne 207 verdichtet und in einem Wärmeübertrager 205 auf eine Eintrittstemperatur 206 gekühlt. Es durchströmt die Absorbtionskolonne 207 von unten nach oben. Im Gegenstrom strömt eine Waschflüssigkeit 232 (Wasser oder ein anderes geeignetes Lösungsmittel). Der Massenstrom eines Produktgases 210 wird gemessen 211. An einem Produktgas-Drosselventil 212 wird der Druck der Absorbtionskolonne 207 eingestellt. Das Produktgas 210 muss nur noch getrocknet werden und kann dann weiter verwertet werden. Die Waschflüssigkeit wird an einem Sumpf 215 einer Kolonne 215 zu einem Flash-Kessel 217 geleitet und dort an einem Drosselventil 216 entspannt. Gelöstes Methan entweicht als Rezirkulationsgas 219 und wird dem vorverdichteten Rohbiogas 201 gemischt. Ein mit einem Durchflussmessgerät 220 gemessener Rezirkulationsstrom ist eine wichtige Größe bei der Optimierung der Anlage. Eine entgaste Waschflüssigkeit 221 wird zu einer Desorbtionskolonne 225 geleitet. Zuvor wird die Temperatur der Waschflüssigkeit an einem Wärmeübertrager 222 eingestellt und mit einem Thermometer 223 überwacht. In die Desorbtionskolonne 225 wird Strip-Luft 234 mit einem Strip-Luft-Gebläse 233 eingeblasen. In der Desorbtionskolonne 225 wird das zugeführte Waschwasser bzw. die zugeführte Waschflüssigkeit 221 im Gegenstrom von $CO_2$ und Teilen von Methan befreit. Ein Schwachgas 236 liegt hier verdünnt mit Luft vor. Es wird auf seine Zusammensetzung analysiert 237 und der Massenstrom mit einem Durchflussmessgerät 235 gemessen. Das Waschwasser 226 wird mit einer Unwälzpumpe 228 zum Kopf der Absorbtionskolonne 207 gefördert und ihr zugeführt 232. Ein Teil des Waschwassers wird als Purge 230 aus dem System entfernt und gegen Frischwasser 227 ersetzt. Durch die Zugabe einer Lauge 231 kann die Selektivität verbessert werden. Als Lauge bieten sich Verbindungen mit $Na^+$ oder $K^+$ als Kationen an.

Gemäß der Erfindung identifiziert man zunächst die unabhängigen Steuerparameter im System. Sie sind:

- Druck 218 im Flash-Kessel 217, eingestellt durch die Drehzahl des Vorverdichters 202;
- Druck 209 in der Absorbtionskolonne 207, eingestellt am Drosselventil 212 im Produktgasstrom 214;
- Wasser-Kreislaufstrom 229, eingestellt an der Umwälzpumpe 228;
- Gas-Eintrittstemperatur 206 an der Absorbtionskolonne 207, eingestellt am Gas-Wärmeübertrager 205;
- Wasser-Eintrittstemperatur 223 an der Desorbtionskolonne 225, eingestellt über den Wasser-Wärmeübertrager 222;
- Luft-Strom 235 durch die Desorbtionskolonne, eingestellt durch die Drehzahl des Strip-Luft-Gebläses 233;
- Lauge-Dosierung 231 in den Umwälzstrom 232, eingestellt über den pH-Wert im Sumpf der Absorbtionskolonne 207;
- Purgestrom 230, eingestellt über die Ausschleusezeit der Umwälzpumpe 228;
- Der Produktgasstrom 211, eingestellt durch die Drehzahl des Hauptverdichters 204.

Weitere Steuerparameter sind:

- Anzahl Gleichgewichtsstufen in der Absorbtionskolonne 207
- Anzahl Gleichgewichtsstufen in der Desorbtionskolonne 225

Sie können nur bei der Auslegung der Anlage variiert werden und sind im Betrieb der Anlage fest vorgegeben.

Nicht alle Aktoren beeinflussen sinnvolle Freiheitsgrade bzw. Steuerparameter. Die Füllstände im Sumpf der Absorbtionskolonne 207, im Flash-Kessel 217 und in der Desorbtionskolonne 225 werden über die Frischwasserzufuhr 227 sowie die Drosselventile 216, 224 geregelt. Sie tragen aber nicht zur Optimierung der Anlage bei.

Die Qualitätsparameter sind

- $CO_2$-Gehalt Produktgas 213;
- Methan-Schlupf (Stoffstrom Methan 237 im Schwachgas 235 geteilt durch den Stoffstrom Methan im Rohbiogas 201)
- Wasserqualität (z.B. pH-Wert, 208)

Die zu minimierende Zielparameter sind die Betriebskosten. Sie setzen sich aus der den Stromkosten (Leistungsaufnahme mal Kosten pro kWh), den Kosten für die Lauge und den Wasserkosten zusammen.

Der Produktgasvolumenstrom 211 wird direkt durch den Betreiber vorgegeben und mit einem PID-Regler geregelt. Es verbleiben acht unabhängige Steuerparameter, die zur Einhaltung von drei Qualitätsparametern und zur Optimierung der Betriebskosten dienen.

Es werden nacheinander alle acht unabhängigen Steuerparameter ausgelenkt und anschließend ein Schritt berechnet, bei dem die Qualitätsbedingungen nicht verändert werden. Der Vorgang wird wiederholt, bis die Anlage kostenoptimiert ist.

Bezugszeichenliste

| | | | |
|---|---|---|---|
| 1 | Einlass | 203 | Gemisch aus Rohbiogas und Rezirkulationsgas |
| 2 | Erster Auslass | | |
| 3 | Zweiter Auslass | 204 | Hauptverdichter |
| 4 | Leitung | 205 | Wärmeübertrager |
| 5 | Erste Membranstufe | 206 | Thermometer |
| 6 | Leitung | 207 | Absorptionskolonne |
| 7 | Zweite Membranstufe | 208 | pH-Wert-Messeinrichtung |
| 8 | Leitung | 209 | Manometer |
| 9 | Dritte Membranstufe | 210 | Produktgas |
| 10 | Leitung | 211 | Durchflussmessgerät |
| 11 | Leitung | 212 | Drosselventil |
| 12 | Leitung | 214 | Produktgasstrom |
| 13 | Kompressor | 215 | Kolonnensumpf |
| 14 | Wärmetauscher | 216 | Drosselventil |
| 15 | Thermometer | 217 | Flash-Kessel |
| 16 | Wärmetauscher | 218 | Manometer |
| 17 | Thermometer | 219 | Rezirkulationsgas |
| 18 | Wärmetauscher | 220 | Durchflussmessgerät |
| 19 | Thermometer | 221 | Waschflüssigkeit |
| 20 | Manometer | 222 | Wärmeübertrager |
| 21 | Vakuumpumpe | 223 | Thermometer |
| 22 | Manometer | 224 | Drosselventil |
| 23 | Drosselventil | 225 | Desorbtionskolonne |
| 24 | Gasflussmessgerät | 226 | Waschwasser |
| 25 | Manometer | 227 | Frischwasser |
| 26 | Drosselventil | 228 | Umwälzpumpe |
| 27 | Messgerät Gaszusammensetzung | 229 | Wasser-Kreisluftstrom |
| 28 | Messgerät Gaszusammensetzung | 230 | Purgestrom |
| 101 | Rohbiogas | 231 | Lauge |
| 103 | Feed-Gas | 232 | Zuführung |
| 108 | Retentat erste Stufe | 233 | Strip-Luft-Gebläse |
| 110 | Temperaturmessung | 234 | Strip-Luft |
| 112 | Produktgas | 235 | Durchflussmessgerät |
| 115 | Drossel | 236 | Schwachgas |
| 119 | Temperaturmessung | 237 | Zusammensetzungsanalyseeinrichtung |
| 121 | Retentat dritte Stufe | | |
| 124 | Schwachgas | | |
| 126 | Permeat zweite Stufe | | |
| 128 | Vakuumpumpe | | |
| 201 | Rohbiogas | | |
| 202 | Vorverdichter | | |

**Patentansprüche**

1. Verfahren zum Betreiben einer Biogasanlage, wobei eine schrittweise Optimierung der Anlage bezüglich eines Zielparameters dadurch erfolgt, dass in einem Optimierungsschritt nach einem Gradienten-basierten Verfahren, zumindest ein Steuerparameter ausgelenkt wird, und die durch die Auslenkung verursachte Änderung des Zielparameters gemessen wird, und in Abhängigkeit der gemessenen Änderung des Zielparameters ein Optimierungsschritt berechnet wird, bei dem Werte für einstellbare Steuerparameter derart bestimmt werden, dass durch Einstellen der berechneten Steuerparameter an auf diese Werte der Zielparameter der Biogasanlage in Richtung eines vorgegebenen Zieles optimiert wird, und die berechneten Werte der Steuerparameter an der Biogasanlage eingestellt werden, und

dieser Optimierungsschritt mehrfach wiederholt wird.

2. Verfahren nach Anspruch 1,
**dadurch gekennzeichnet**
**dass** bei jedem Optimierungsschritt mehrere Steuerparameter ausgelenkt werden, und/ oder dass die Steuerparameter von einer Steuereinrichtung gesteuert selbsttätig mittels entsprechender Stellelemente ausgelenkt, berechnet und an den entsprechenden Stellelementen eingestellt werden.

3. Verfahren nach einem der Ansprüche 1 oder 2,
**dadurch gekennzeichnet**
**dass** nach einer Auslenkung eines Steuerparameters neben dem Zielparameter auch zumindest ein Qualitätsparameter gemessen wird, wobei der Messwert des Qualitätsparameters zur Einhaltung einer Nebenbedingung verwendet wird.

4. Verfahren nach Anspruch 3,
**dadurch gekennzeichnet**
**dass** die Nebenbedingung mittels einer Regeleinrichtung, wie z.B. einem P-Regler, einem I-Regler, einem D-Regler oder einer beliebigen Kombination dieser Regler, geregelt wird, wobei für das Regeln eines jeden Qualitätsparameters ein Steuerparameter verwendet wird, der mittels der Regeleinrichtung in Abhängigkeit von dem gemessenen Wert des Qualitätsparameters eingestellt wird, oder
**dass** die Nebenbedingungen bei der Berechnung des Optimierungsschrittes derart mit berücksichtigt werden, dass der Qualitätsparameter einen bestimmten Wert einhält oder einen entsprechenden Schwellwert nicht ober- oder unterschreitet.

5. Verfahren nach einem der Ansprüche 1 bis 4,
**dadurch gekennzeichnet**
**dass** mit entsprechenden Sensoren die Steuerparameter gemessen werden und die Stellelemente nach Maßgabe der entsprechenden Messwerte betätigt werden, um die bei den Optimierungsschritten berechneten Werte für die Steuerparameter einzustellen und/oder dass die der Optimierungsschritt wiederholt wird bis keine oder nur eine vorbestimmte geringe Änderung des Zielparameters bei einem Optimierungsschritt erfolgt.

6. Verfahren nach einem der vorhergehenden Ansprüche, **dadurch gekennzeichnet, dass** bei jedem Optimierungsschritt die partiellen Ableitungen des Zielparameters nach den Steuerparametern bestimmt werden und die partiellen Ableitungen zur Berechnung der Steuerparameter verwendet werden.

7. Verfahren nach einem der vorhergehenden Ansprüche, **dadurch gekennzeichnet, dass** der Optimierungsschritt einem Optimierungsverfahren nach dem Gradientenabstiegsverfahren, dem Gradientenabstiegsverfahren mit Nebenbedingungen oder dem Quasi-Newton-Verfahren entspricht, oder dass der Optimierungsschritt ein nicht-Gradienten-basierten Algorithmus entspricht..

8. Verfahren nach einem der vorhergehenden Ansprüche, **dadurch gekennzeichnet, dass** zunächst alle verfügbaren Steuerparameter ausgelenkt und in den nachfolgenden Optimierungsschritten nur eine kleinere Auswahl an Steuerparametern ausgelenkt werden, wobei vorzugsweise nach einer vordefinierten Anzahl an Optimierungsschritten nur eine reduzierte Anzahl Steuerparameter ausgelenkt werden.

9. Verfahren nach Anspruch 8,
**dadurch gekennzeichnet**
**dass** nachdem ein Optimierungsschritt, bei dem eine reduzierte Anzahl Steuerparameter ausgelenkt wird, nur eine geringe Annäherung des Zielparameters in Richtung des vorgegebenen Ziels ergibt, wieder mehrere oder alle verfügbaren Steuerparameter ausgelenkt und erneut geprüft wird, ob eine stärkere Veränderung des Zielparameters möglich ist.

10. Verfahren nach einem der vorhergehenden Ansprüche, **dadurch gekennzeichnet, dass** mehrere Steuerparameter mittels einer Hauptkomponentenanalyse zu kombinierten Steuerparametern gebündelt werden, welche Linearkombinationen und nicht-lineare Kombinationen der einzelnen Steuerparameter sind, wodurch die Anzahl der veränderbaren Steuerparameter abnimmt und die Wiederholung der Optimierungsschritte schnell zum Ziel geführt wird.

11. Verfahren nach einem der vorhergehenden Ansprüche, **dadurch gekennzeichnet, dass** die Biogasaufbereitungsanlage nach dem Verfahren der membranbasierten Gaspermeation, der Druckwasserwäsche, der organischen Wäsche oder der Druckwechseladsorption arbeitet, und/oder, dass die Biogasaufbereitungsanlage mit einem Power to Gas-Modul gekoppelt wird, wobei das Rohbiogas mit Wasserstoff vermischt wird, der auf biologischen oder katalytischem Wege mit dem $CO_2$ des Rohbiogases zu Methan reagiert und das so erzeugte Rohbiogas mit erhöhtem Methangehalt einer Biogasaufbereitungsanlage zugeführt wird, und/oder, dass das Verfahren zum Betreiben einer Biogasanlage ein Gasaufbereitungsverfahren umfasst, wobei als Gasaufbereitungsverfahren das Gaspermeationsverfahren verwendet wird, wobei ein Teil der Membranen mit einstellbaren Ventilen versehen sind, so dass die Module einzelnen Stufen zuordbar sind, wobei die Regelung auch die Membranflächen im laufenden Betrieb ändert.

12. Biogasanlage umfassend mehrere Komponenten zum Vergären von Substraten und/oder Aufbereiten von Gas, wobei die Funktionsweise der Komponenten von Steuerparametern abhängt, welche mittels entsprechender Stellelemente einstellbar sind, wobei die Biogasanlage eine Steuereinrichtung aufweist, die zum Ausführen eines der Verfahren nach Anspruch 1 bis 11 ausgebildet ist.

13. Biogasanlage nach Anspruch 12,
**dadurch gekennzeichnet,**
**dass** die Stellelemente eines oder mehrere der folgenden Stellelemente aufweist:

- Temperiereinrichtung, wie z.B. Wärmetauscher, Heizeinrichtung oder Kühleinrichtung,
- Drucksteuereinrichtung, wie z.B. Pumpe, Drosselventil,
- Antrieb für eine Rühr- oder Rütteleinrichtung,
- Antrieb für ein Gebläse.

14. Biogasanlage nach Anspruch 13,
**dadurch gekennzeichnet,**
**dass** den Stellelementen jeweils ein Sensor zugordnet ist, mit welchen der mit den Stellelementen beeinflussbare Steuerparameter messbar ist.

15. Biogasanlage nach einem der Ansprüche 12 bis 14,
**dadurch gekennzeichnet,**
**dass** die Biogasanlage eine Membranfiltereinrichtung mit mehreren Membraneinheiten aufweist, die einzeln zu- oder abschaltbar sind, wobei in einer Zuleitung zur Membranfiltereinrichtung eine Temperiereinrichtung und ein Thermometer zum Einstellen der Temperatur des zur Membranfiltereinrichtung zugeführten Gases und in zumindest einer Ableitung von der Membranfiltereinrichtung eine Drucksteuereinrichtung zum Einstellen eines Retentat- oder Permeatdruckes und ein Manometer zum Messen dieses Druckes vorgesehen sind.

**Claims**

1. Method for operating a biogas plant, wherein the plant is optimized step by step with respect to a target parameter by an optimizing step which involves deflecting at least one control parameter in accordance with a gradient-based method and measuring the change in the target parameter caused by the deflection, and, depending on the measured change in the target parameter, calculating an optimizing step in which values for adjustable control parameters are determined in such a manner that, by setting the calculated control parameters to these values, the target parameter of the biogas plant is optimized towards a prescribed target and the calculated values of the control parameters are set on the biogas plant, and this optimizing step is repeated a number of times.

2. Method according to claim 1, **characterized in that** during each optimizing step several control parameters are deflected, and/or that the control parameters controlled by a control means are automatically deflected, calculated and set at the appropriate adjusting elements by means of appropriate adjusting elements.

3. Method according to any of claims 1 or 2 **characterized in that** after the deflection of a control parameter also at least one quality parameter is measured in addition to the target parameter, with the measured value of the quality parameter being used to comply with a constraint.

4. Method according to claim 3, **characterized in that** the constraint is regulated by means of a regulating device such as a P regulator, an I regulator, a D regulator or any desired combination of said regulators, wherein for the regulation of each quality parameter a control parameter is used which by means of the regulating device is adjusted depending on the measured value of the quality parameter, or that the constraints in calculating the optimizing step are taken into account in such a manner that the quality parameter maintains a certain value or does not exceed or fall below a corresponding threshold value.

5. Method according to any of claims 1 to 4, **characterized in that** the control parameters are measured with appropriate sensors and the adjusting elements are activated in accordance with the corresponding measured values in order to set the values for the control parameters calculated during the optimizing steps, and/or that the optimizing step is repeated until no change or only a predetermined small change in the target parameter is effected during an optimizing step.

6. Method according to any of the preceding claims, **characterized in that** during each optimizing step the partial deflections of the target parameter are determined according to the control parameters and the partial deflections are used to calculate the control parameters.

7. Method according to one of the preceding claims, **characterized in that** the optimizing step corresponds to an optimizing method according to the gradient descent method, the gradient descent method with constraints, or the quasi-Newton method, or that the optimizing step corresponds to a non-gradient-based algorithm.

8. Method according to any of the preceding claims, **characterized in that** initially all the available control parameters are deflected and in the successive optimizing steps only a smaller selection of control parameters are deflected, wherein, preferably after a predefined number of optimizing steps, only a reduced number of control parameters are deflected.

9. Method according to claim 8, **characterized in that** since an optimizing step in which a reduced number of control parameters are deflected only produces a low approximation of the target parameter towards the specified target, several or all available control parameters are again deflected and re-examined as to whether a greater change of the target parameter is possible.

10. Method according to any of the preceding claims, **characterized in that** several control parameters are bundled by means of a principal component analysis into combined control parameters which are linear combinations and non-linear combinations of the individual control parameters, by means of which the number of alterable control parameters is reduced and the objective of repeating the optimizing steps is quickly achieved.

11. Method according to any of the preceding claims, **characterized in that** the biogas treatment plant operates according to the method of membrane-based gas permeation, pressurized water scrubbing, organic scrubbing or pressure swing adsorption, and/or that the biogas treatment plant is coupled to a power-to-gas module, wherein the raw biogas is mixed with hydrogen, which by biological or catalytic action with the $CO_2$ of the raw biogas reacts to methane and the raw biogas with an increased methane content thus produced is fed to a biogas treatment plant, and/or that the method for operating a biogas plant comprises a gas treatment method wherein the gas permeation method is used as a gas treatment method, wherein some of the membranes are provided with adjustable valves so that the modules can be assigned to individual steps, with the regulation also changing the membrane surfaces during operation.

12. Biogas plant comprising several components for fermenting substrates and/or treating gas, wherein the operating mode of the components depends on control parameters which are adjustable by means of appropriate adjusting elements, wherein the biogas plant has a control means designed to carry out one of the methods according to claim 1 to 11.

13. Biogas plant according to claim 12, **characterized in that** the adjusting elements feature one or more of the following adjusting elements:

   - Temperature control means, e.g. heat exchanger, heating device or cooling device,
   - Pressure control device, e.g. pump, throttle valve,
   - Drive for a stirring or agitating device,
   - Drive for a blower.

14. Biogas plant according to claim 13, **characterized in that** to each adjusting element is assigned a sensor with which can be measured the control parameter influenced by the adjusting elements.

15. Biogas plant according to any of claims 12 to 14, **characterized in that** the biogas plant has a membrane filtration device with several membrane units, which can be individually switched on or off, wherein in a supply line to the membrane filtration device is provided a temperature control device and a thermometer for adjusting the temperature of the gas fed to the membrane filtration device, and in at least one discharge line from the membrane filtration device are provided a pressure control device for setting a retentate or permeate pressure and a manometer for measuring this pressure.

**Revendications**

1. Procédé d'exploitation d'un système biogaz, une optimisation par étapes du système concernant un paramètre cible étant réalisée de telle sorte que, lors d'une étape d'optimisation selon un procédé se basant sur des gradients, au moins un paramètre de commande est dévié, et la modification du paramètre cible causée par la déviation est mesurée, et une étape d'optimisation est calculée en fonction de la modification mesurée du paramètre cible, étape d'optimisation pendant laquelle des valeurs pour les paramètres de commande réglables sont déterminées de telle sorte que le paramètre cible du système biogaz est optimisé en direction d'une cible prescrite par le réglage des paramètres de commande calculés sur ces valeurs, et les valeurs calculées des paramètres de commande sont réglés sur le système biogaz, et cette étape d'optimisation est répétée plusieurs fois.

2. Procédé selon la revendication 1, **caractérisé en ce que** plusieurs paramètres de commande sont déviés à chaque étape d'optimisation, et/ou **en ce que** les paramètres de commande sont commandés par une unité de commande, déviés automatiquement au moyen d'éléments de réglage correspondants, calculés et réglés sur les éléments de réglage correspondants.

3. Procédé selon l'une des revendications 1 ou 2, **caractérisé en ce qu'**au moins un paramètre de qualité est également mesuré après une déviation d'un paramètre de commande à côté du paramètre cible, la valeur mesurée du paramètre de qualité étant utilisée pour observer une contrainte.

4. Procédé selon la revendication 3, **caractérisé en ce que** la contrainte est réglée au moyen d'un dispositif de régulation tel qu'un régulateur P, un régulateur I, un régulateur D ou une quelconque combinaison de ces régulateurs, un paramètre de commande étant utilisé pour la régulation d'un paramètre de qualité, paramètre de commande qui est réglé au moyen du dispositif de régulation en fonction de la valeur mesurée du paramètre de qualité, ou **en ce que** les contraintes lors du calcul de l'étape d'optimisation sont prises en considération de telle sorte que le paramètre de qualité respecte une valeur déterminée ou ne dépasse pas une valeur seuil correspondante par le haut ou par le bas.

5. Procédé selon l'une quelconque des revendications 1 à 4, **caractérisé en ce que** les paramètres de commande sont mesurés avec des capteurs correspondants et les éléments de réglage sont actionnés selon les valeurs mesurées correspondantes afin de régler les valeurs calculées pour les paramètres de commande lors des étapes d'optimisation et/ou **en ce que** l'étape d'optimisation est répétée jusqu'à ce que plus aucune ou qu'une légère modification prédéterminée seulement du paramètre cible ne soit ou soit effectuée lors d'une étape d'optimisation.

6. Procédé selon l'une des revendications précédentes, **caractérisé en ce que** les dérivations partielles du paramètre cible sont déterminées selon les paramètres de commande et les dérivations partielles sont utilisées pour calculer les paramètres de commande lors de chaque étape d'optimisation.

7. Procédé selon l'une des revendications précédentes, **caractérisé en ce que** l'étape d'optimisation correspond à un procédé d'optimisation selon l'algorithme du gradient, à l'algorithme du gradient avec des contraintes ou à la méthode quasi-Newton, ou **en ce que** l'étape d'optimisation correspond à un algorithme non basé sur le gradient.

8. Procédé selon l'une des revendications précédentes, **caractérisé en ce que** dans un premier temps tous les paramètres de commande disponibles sont déviés et seulement une petite sélection de paramètres de commande est déviée dans les étapes d'optimisation suivantes, seul un nombre réduit de paramètres de commande étant déviés de préférence selon un nombre prédéterminé d'étapes d'optimisation.

9. Procédé selon la revendication 8, **caractérisé en ce qu'**après qu'une étape d'optimisation, lors de laquelle un nombre réduit de paramètres de commande est dévié, a donné un petit rapprochement du paramètre cible en direction de la cible définie seulement, les paramètres de commande disponibles en tout ou partie sont déviés de nouveau et recontrôlés si une modification plus importante du paramètre cible est possible.

10. Procédé selon l'une des revendications précédentes, **caractérisé en ce que** plusieurs paramètres de commande sont mis en commun avec des paramètres de commande combinés au moyen d'une analyse de composants principaux, paramètres de commande combinés qui sont des combinaisons linéaires et des combinaisons non linéaires des différents paramètres de commande diminuant le nombre des paramètres de commande modifiables et permettant rapidement de répéter les étapes d'optimisation.

11. Procédé selon l'une des revendications précédentes, **caractérisé en ce que** le système de traitement de biogaz fonctionne selon le procédé de perméation des gaz à membranes, de lavage sous pression, de lavage organique ou d'adsorption à modulation de pression, et/ou **en ce que** le système de traitement de biogaz est couplé à un module Power to Gas, le biogaz brut étant mélangé à de l'hydrogène qui réagit au méthane avec du $CO_2$ par voie catalytique ou biologique, et le biogaz brut ainsi généré avec une teneur élevée en méthane est amené à un système de traitement de biogaz, et/ou **en ce que** le procédé d'exploitation d'un système biogaz comprend un procédé de traitement de gaz, la perméation des gaz étant utilisée comme procédé de traitement de gaz, une partie des membranes étant prévue avec des vannes réglables de telle sorte que les modules soient attribuables aux différentes étapes, la régulation modifiant également les surfaces de membranes durant le fonctionnement.

12. Système biogaz comprenant plusieurs composants pour la fermentation de substrats et/ou le traitement de gaz, le fonctionnement des composants dépend de paramètres de commande qui sont réglables au moyen d'éléments de réglage correspondants, le système biogaz comprenant une unité de commande qui est formée pour l'exécution d'un des procédés selon les revendications 1 à 11.

13. Système biogaz selon la revendication 12, **caractérisé en ce que** les éléments de réglage présentent un ou plusieurs des éléments de réglage suivants :

   - dispositif de régulation tel qu'un échangeur thermique, un dispositif de chauffage ou un dispositif de refroidissement,
   - dispositif de commande de pression tel qu'une pompe, une vanne d'étranglement,
   - entraînement pour un vibreur ou agitateur,
   - entraînement pour une soufflante.

14. Système biogaz selon la revendication 13, **caractérisé en ce qu'**un capteur est attribué respectivement aux éléments de réglage avec lesquels le paramètre de commande influençable avec les éléments de réglage peut être mesuré.

15. Système biogaz selon l'une quelconque des revendications 12 à 14, **caractérisé en ce que** le système biogaz présente un dispositif de filtrage à membranes avec plusieurs unités à membranes qui peuvent être enclenchées et déclenchées individuellement, un dispositif de régulation et un thermomètre pour le réglage de la température du gaz alimenté au dispositif de filtrage à membranes étant prévus dans une conduite amenant au dispositif de filtrage à membranes et un dispositif de commande de pression étant prévu dans au moins une dérivation du dispositif de filtrage à membranes pour régler la pression du perméat ou du rétentat et un manomètre pour mesurer cette pression étant prévu.

# Figur 1

# Figur 2

EP 3 494 204 B1

| | | Startwert | 1. Schritt | 2. Schritt | 3. Schritt | 4. Schritt | 5. Schritt | 6. Schritt | 7. Schritt | 8. Schritt | 9. Schritt | 10. Schritt | 11. Schritt | 12. Schritt | 13. Schritt | 14. Schritt | 15. Schritt | 16. Schritt | Kontrolle 1 | Kontrolle 2 | Kontrolle 3 | 17. Schritt |
|---|---|---|---|---|---|---|---|---|---|---|---|---|---|---|---|---|---|---|---|---|---|---|
| $p_{R2}$ | [bar] | 17,00 | 16,68 | 16,46 | 16,24 | 15,99 | 15,73 | 15,45 | 15,15 | 14,83 | 14,47 | 14,43 | 14,51 | 13,95 | 13,98 | 13,73 | 13,55 | 13,51 | 13,23 | 13,60 | 13,57 | 13,90 |
| $p_{P2}$ | [bar] | 1,05 | 0,81 | 0,78 | 0,75 | 0,71 | 0,68 | 0,64 | 0,60 | 0,56 | **0,50** | **0,50** | **0,50** | **0,50** | **0,50** | **0,50** | **0,50** | **0,50** | 0,46 | **0,50** | **0,50** | **0,50** |
| $p_{R3}$ | [bar] | 2,90 | 2,89 | 2,88 | 2,88 | 2,88 | 2,88 | 2,88 | 2,87 | 2,87 | 2,87 | 2,86 | 2,84 | 2,87 | 2,96 | 3,10 | 3,20 | 3,23 | 3,22 | 3,21 | 3,20 | 3,13 |
| $T_1$ | [°C] | 25,00 | 24,87 | 24,78 | 24,70 | 24,60 | 24,50 | 24,40 | 24,29 | 24,17 | 24,05 | 19,91 | **10,00** | **10,00** | **10,00** | **10,00** | **10,00** | **10,00** | 9,94 | 3,83 | **10,00** | **10,00** |
| $T_2$ | [°C] | 28,00 | 27,93 | 27,89 | 27,84 | 27,79 | 27,74 | 27,69 | 27,63 | 27,57 | 27,50 | 25,15 | 20,68 | **45,00** | **45,00** | **45,00** | **45,00** | **45,00** | 44,93 | 37,66 | 39,36 | 17,44 |
| $T_3$ | [°C] | 25,00 | 25,01 | 25,02 | 25,03 | 25,04 | 25,05 | 25,06 | 25,07 | 25,08 | 25,09 | 25,51 | 26,30 | 22,45 | **10,00** | **10,00** | **10,00** | **10,00** | 10,01 | 10,80 | 13,67 | 16,27 |
| $n_2$ | [Stück] | 23,00 | 23,00 | 23,00 | 23,00 | 23,00 | 23,00 | 23,00 | 23,00 | 23,00 | 23,00 | 23,00 | 22,99 | 23,03 | 23,29 | 24,67 | 25,33 | 25,60 | 25,60 | 25,57 | 25,57 | 25,13 |
| $n_3$ | [Stück] | 29,00 | 29,00 | 29,00 | 29,00 | 29,00 | 29,00 | 29,01 | 29,00 | 29,01 | 29,01 | 29,04 | 29,10 | 28,83 | 28,22 | 26,10 | 24,97 | 24,61 | 24,61 | 24,65 | 24,66 | 25,38 |
| $n_1$ | [Stück] | 18,00 | 18,00 | 18,00 | 18,00 | 18,00 | 18,00 | 17,99 | 18,00 | 17,99 | 17,99 | 17,96 | 17,90 | 18,13 | 18,50 | 19,24 | 19,69 | 19,80 | 19,79 | 19,78 | 19,77 | 19,49 |
| $x_{CO2,PG}$ | [%] | | 1,41% | 1,01% | 1,00% | 1,00% | 1,00% | 1,00% | 1,00% | 1,00% | 1,00% | 0,98% | 1,00% | 1,00% | 1,04% | 1,01% | 0,99% | 1,01% | 1,00% | 1,00% | 1,00% | 1,00% | 1,06% |
| $x_{CH4,SG}$ | [%] | | 0,74% | 0,70% | 0,70% | 0,70% | 0,70% | 0,70% | 0,70% | 0,70% | 0,70% | 0,70% | 0,71% | 0,70% | 0,70% | 0,67% | 0,70% | 0,70% | 0,70% | 0,70% | 0,70% | 0,67% | 0,70% |
| $P$ | [kW] | **215,6** | **217,4** | **215,7** | **213,8** | **211,8** | **209,8** | **207,8** | **205,8** | **203,9** | **202,4** | **200,3** | **197,3** | **196,5** | **196,3** | **196,1** | **195,0** | **195,1** | **194,7** | **193,1** | **194,7** | **192,3** |
| $\Delta P_{soll}$ | [kW] | | -2,00 | -2,00 | -2,00 | -2,00 | -2,00 | -2,00 | -2,00 | -2,00 | -2,00 | -2,00 | -2,00 | -2,00 | -2,00 | -2,00 | -0,10 | -0,50 | -2,00 | -2,00 | -2,00 | -2,00 |
| $\Delta P_{ist}$ | [kW] | | 1,81 | -1,70 | -1,96 | -2,00 | -1,99 | -1,98 | -1,96 | -1,95 | -1,47 | -2,14 | -2,98 | -0,78 | -0,19 | -0,26 | -1,00 | 0,10 | -0,40 | -1,68 | 1,66 | -2,38 |

# Figur 3

# Figur 4

EP 3 494 204 B1

| | | Start | 1. Schritt | 2. Schritt | 3. Schritt | 4. Schritt | 5. Schritt | 6. Schritt |
|---|---|---|---|---|---|---|---|---|
| Feedgas | [Nm³/h] | 1178 | 1185.3 | 1187.3 | 1192.4 | 1199.5 | 1189.4 | 1190.7 |
| $p_{R2}$ | [bar] | 12.12 | 11.52 | 11.61 | 11.59 | 11.62 | 11.57 | 11.58 |
| $p_{P2}$ | [bar] | 0.60 | 0.43 | 0.51 | 0.49 | 0.49 | 0.49 | 0.49 |
| $P_{R3}$ | [bar] | 2.22 | 2.21 | 2.22 | 2.23 | 2.25 | 2.22 | 2.22 |
| $T_1$ | [°C] | 40 | 40 | 40 | 40 | 40 | 40 | 40 |
| $T_2$ | [°C] | 40 | 40 | 40 | 40 | 40 | 40 | 40 |
| $T_3$ | [°C] | 40 | 40 | 40 | 40 | 40 | 40 | 40 |
| Produktgas | [Nm³/h] | 750.4 | 754.6 | 756.7 | 760.0 | 764.1 | 757.9 | 758.8 |
| $x_{CO2,PG}$ | [mol/mol] | 0.99% | 0.89% | 1.01% | 1.01% | 0.98% | 1.00% | 1.00% |
| $x_{CH4,SG}$ | [mol/mol] | 0.54% | 0.48% | 0.50% | 0.48% | 0.53% | 0.50% | 0.49% |
| $P_{Kompressor}$ | [kW] | 255.6 | 251.0 | 249.9 | 251.6 | 248.0 | 249.7 | 250.8 |

Figur 5

EP 3 494 204 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2012000727 A1
- DE 102013004079 A1
- WO 2014075850 A1
- WO 20122012175701 A1
- DE 102006053863 A1